# EUROPEAN PATENT SPECIFICATION

(11) **EP 4 051 177 B1**
(45) Date of publication and mention of the grant of the patent: **18.12.2024**
(21) Application number: 20804032.9
(22) Date of filing: 14.10.2020
(51) Int. Cl.: A61F 2/24, A61F 2/958

(54) **BLOW MOLDED BALLOON SHOULDER ASSEMBLY FOR A TRANSCATHETER DELIVERY DEVICE**
BLASGEFORMTE BALLONSCHULTERANORDNUNG FÜR EINE TRANSKATHETERFREISETZUNGSVORRICHTUNG
ENSEMBLE ÉPAULEMENT DE BALLONNET MOULÉ PAR SOUFFLAGE POUR DISPOSITIF DE POSE PAR TRANSCATHÉTER

(30) Priority: 31.10.2019 US 201962928951 P
(43) Date of publication of application: 07.09.2022
(73) Proprietor: Edwards Lifesciences Corporation, Irvine, CA 92614-5688 (US)
(72) Inventor: ZHU, Yidong M., Irvine, CA 92614 (US)
(74) Representative: Eisenführ Speiser
(86) International application number: PCT/US2020/055520
(87) International publication number: WO 2021/086608

(56) References cited:
- EP-A2- 1 802 368
- EP-B1- 1 802 368
- US-A1- 2005 228 429
- US-A1- 2011 077 731
- US-A1- 2012 046 739
- US-A1- 2018 214 289

## Description

### CROSS REFERENCE TO RELATED APPLICATION

This application claims the benefit of U.S. Provisional Application Ser. No. 62/928,951 entitled "BLOW MOLDED BALLOON SHOULDER ASSEMBLY FOR A TRANSCATHETER DELIVERY DEVICE," filed October 31, 2019,

### FIELD

The present disclosure concerns embodiments of a blow molded balloon shoulder assembly for a balloon catheter for implantation of a medical device, such as a prosthetic heart valve.

### BACKGROUND

Endovascular delivery devices are used in various procedures to deliver prosthetic medical devices or instruments to locations inside the body that are not readily accessible by surgery or where access without surgery is desirable. Access to a target location inside the body can be achieved by inserting and guiding the delivery device through a pathway or lumen in the body, including, but not limited to, a blood vessel, an esophagus, a trachea, any portion of the gastrointestinal tract, a lymphatic vessel, to name a few. In one specific example, a prosthetic heart valve can be mounted in a crimped state on the distal end of a delivery device and advanced through the patient's vasculature (e.g., through a femoral artery and the aorta) until the prosthetic valve reaches the implantation site in the heart. The prosthetic valve is then expanded to its functional size such as by inflating a balloon on which the prosthetic valve is mounted, or by deploying the prosthetic valve from a sheath of the delivery device so that the prosthetic valve can self-expand to its functional size.

Balloon-expandable prosthetic valves may be preferred for replacing calcified native valves because the catheter balloon can apply a sufficient expansion force to expand and anchor the frame of the prosthetic valve against the surrounding calcified tissue. In one known technique for delivering a prosthetic heart valve, the prosthetic heart valve may be crimped on a valve retaining portion of a balloon of the delivery catheter prior to insertion into the patient's body. Examples of such delivery catheters are disclosed in: US 2018/0214289 A1; EP 1 802 368 A2; US 2012/0046739 A1; US 2005/0228429 A1; US 2011/0077731 A1. The valve retaining portion may be formed by a distal balloon shoulder and proximal distal shoulder arranged inside the balloon and mounted on shafts of the delivery catheter. The distal and proximal balloon shoulders may assist in retaining the prosthetic heart valve on the valve retaining portion of the balloon during delivery through a patient's vasculature. Typically, the balloon shoulders are formed via injection modeling, resulting in hard, inflexible, and thick parts. These characteristics of the balloon shoulders make them difficult to insert into legs of the balloon during manufacturing of a balloon catheter. Additionally, the increased stiffness and thickness of the injection molded balloon shoulders may result in the formation of gaps between the shoulders and the valve during maneuvering the delivery catheter through a sheath of the delivery system, within curves in the patient's vasculature in route to the target treatment site, thereby resulting in potential damage to the sheath. Further still, the increased hardness and size of the injection molded shoulders may result in higher retrieving forces of the delivery catheter through the sheath, post valve deployment. Accordingly, improvements in balloon shoulders of balloon catheters for delivering implantable medical devices, such as prosthetic heart valves, are desirable.

### SUMMARY

Disclosed herein are balloon catheters, balloon shoulder assemblies for balloon catheters, as well as related methods for forming balloon shoulder assemblies and balloon catheters. The balloon catheters can be used to deliver a medical device, tools, agents, or other therapy to a location within a body of a subject. In some embodiments, balloon catheters can be used to deliver an implantable medical device, such as a prosthetic heart valve, to a target site in a patient, such as a heart. In some embodiments, balloon catheters can be a component of a delivery system (e.g., an endovascular or transcatheter delivery system) that can be used to deliver a prosthetic heart valve or other implantable medical device. Balloon catheters can include a balloon shoulder assembly positioned inside a main balloon of the balloon catheter to form a valve retaining portion onto which the prosthetic heart valve (or other implantable medical device, such as a stent) can be crimped. In some embodiments, the balloon shoulder assembly may include a proximal balloon shoulder and a distal balloon shoulder that may be blow molded as one piece with a central connecting portion or blow molded as separate proximal and distal balloon shoulders. Additionally, in some embodiments, the blow molded balloon shoulders may be inflatable with various fluids. In other embodiments, the blow molded balloon shoulders may be non-inflatable, and instead, may be configured to structurally support a balloon that they are placed inside, in a non-inflated state.

In one representative embodiment, a balloon shoulder assembly for a balloon catheter can include a proximal balloon shoulder including a proximal collar portion that extends radially outward from a proximal shaft portion, relative to a central axis of the balloon shoulder assembly; and a distal balloon shoulder including a distal collar portion that extends radially outward from a distal shaft portion. In some embodiments, each of the proximal balloon shoulder and the distal balloon shoulder are hollow and comprise a compressible, blow molded material.

In some embodiments, outer walls of the proximal balloon shoulder and outer walls of the distal balloon shoulder are configured to compress or stretch under applied pressure and return back to an uncompressed or unstretched state upon removal of the applied pressure.

In some embodiments, the proximal collar portion is spaced away from the distal collar portion, in an axial direction relative to the central axis.

In some embodiments, the proximal balloon shoulder and the distal balloon shoulder are molded as one piece with a central connecting portion, the central connecting portion arranged between and connecting the proximal collar portion and the distal collar portion.

In some embodiments, the proximal balloon shoulder further includes a proximal transition portion extending between and connecting the proximal collar portion and the central connecting portion, the proximal transition portion narrowing in diameter from the proximal collar portion to the central connecting portion and the distal balloon shoulder further includes a distal transition portion extending between and connecting the distal collar portion and the central connecting portion, the distal transition portion narrowing in diameter from the distal collar portion to the central connecting portion.

In some embodiments, the proximal balloon shoulder and the distal balloon shoulder are molded separately, as two separate pieces.

In some embodiments, each of the proximal balloon shoulder and the distal balloon shoulder are inflatable.

In some embodiments, the proximal balloon shoulder and the distal balloon shoulder are not fluidly coupled with one another and are individually inflatable.

In some embodiments, each of the proximal shoulder and the distal balloon shoulder are not inflatable.

In some embodiments, each of the proximal balloon shoulder and the distal balloon shoulder have a wall thickness in a range of 0.0001 inch to 0.010 inch. 1 inches to cm equals 2.54 cm, this conversion is applied through the description.

In some embodiments, the proximal collar portion has an open end facing an open end of the distal collar portion.

In some embodiments, each of the proximal collar portion and the distal collar portion are funnel-shaped with a wider end that flares radially outward from a narrower end, where the narrower end of the proximal collar portion is connected to the proximal shaft portion, where the narrower end of the distal collar portion is connected to the distal shaft portion, and where the wider ends of each of the proximal collar portion and the distal collar portion face one another and are arranged normal to the central axis.

In some embodiments, each of the proximal collar portion and the distal collar portion have a bulbous shape with a wider, central portion that narrows to two opposite ends.

In some embodiments, one or more of the proximal collar portion and the distal collar portion includes a central, cylindrical body arranged between a first tapered end portion and a second tapered end portion, the first tapered end portion and the second tapered end portion tapering, in opposite directions, from the central cylindrical body to one of the central connecting portion or a corresponding one of the proximal shaft portion or the distal shaft portion.

In some embodiments, one or more of the proximal collar portion and the distal collar portion includes a curved, central portion positioned between a first tapered end portion and a second tapered end portion, the first tapered end portion and the second tapered end portion tapering, in opposite directions, from the central portion to one of the central connecting portion or a corresponding one of the proximal shaft portion or the distal shaft portion.

In some embodiments, one or more of the proximal collar portion and the distal collar portion includes an elongate tapered portion, a shorter tapered portion, and a central ring portion positioned between the elongate tapered portion and the shorter tapered portion.

In some embodiments, the balloon catheter is part of a transcatheter heart valve delivery system.

In some embodiments, the proximal balloon shoulder and the distal balloon shoulder are configured to be positioned within and support an inflatable, main balloon of the balloon catheter.

In another representative embodiment, a method of manufacturing a balloon catheter can include blow molding a balloon shoulder assembly including a proximal balloon shoulder and a distal balloon shoulder; and installing the blow molded balloon shoulder assembly in the balloon catheter by positioning the balloon shoulder assembly within an inflatable, main balloon of the balloon catheter.

In some embodiments, the proximal balloon shoulder and the distal balloon shoulder are spaced apart from one another, in an axial direction relative to a central axis of the balloon catheter.

In some embodiments, positioning the balloon shoulder assembly within the main balloon forms a device retaining portion on the main balloon, in a space that separates the proximal balloon shoulder and the distal balloon shoulder.

In some embodiments, the device retaining portion is adapted to receive a prosthetic medical device thereon.

In some embodiments, the prosthetic medical device is a prosthetic heart valve.

In some embodiments, blow molding the balloon shoulder assembly includes blow molding the proximal balloon shoulder and the distal balloon shoulder as one piece with a central connecting portion of the balloon shoulder assembly, the central connecting portion arranged between the proximal balloon shoulder and the distal balloon shoulder.

In some embodiments, blow molding the balloon shoulder assembly includes blow molding the proximal balloon shoulder and the distal balloon shoulder as separate pieces.

In some embodiments, blow molding the balloon shoulder assembly includes forming the proximal balloon shoulder and the distal balloon shoulder to be fully inflatable, with ends that are adapted to be sealed around one or more shafts of the balloon catheter during manufacturing and contain pressure therein.

In some embodiments, blow molding the balloon shoulder assembly includes forming each of the proximal balloon shoulder and the distal balloon shoulder to be at least partially open and not inflatable.

In some embodiments, the method can further include mounting the balloon shoulder assembly to one or more shafts of the balloon catheter, prior to positioning the balloon shoulder assembly within the main balloon.

In some embodiments, mounting the balloon shoulder assembly to the one or more shafts of the balloon catheter includes mounting the proximal balloon shoulder to an outer shaft of the balloon catheter and mounting the distal balloon shoulder to an inner shaft of the balloon catheter.

In some embodiments, mounting the balloon shoulder assembly to the one or more shafts of the balloon catheter includes mounting the proximal balloon shoulder to an outer shaft of the balloon catheter and mounting the distal balloon shoulder to a nosecone of the balloon catheter.

In some embodiments, mounting the balloon shoulder assembly to the one or more shafts of the balloon catheter includes mounting the proximal balloon shoulder to an inner shaft of the balloon catheter and mounting the distal balloon shoulder to a nosecone of the balloon catheter.

In some embodiments, mounting the balloon shoulder assembly to the one or more shafts of the balloon catheter includes mounting the proximal balloon shoulder to an inner shaft of the balloon catheter and mounting the distal balloon shoulder to the inner shaft.

In some embodiments, the method can further include inflating the proximal balloon shoulder and the distal balloon shoulder from a deflated state to an inflated state, after positioning the balloon shoulder assembly within the main balloon. In some embodiments, inflating the proximal balloon shoulder and the distal balloon shoulder includes delivering a fluid to an interior of the proximal balloon shoulder and an interior of the distal balloon shoulder. In some embodiments, the fluid includes at least one of saline, a contrast mixture, a biocompatible media, a curable material, and a non-curable material.

In some embodiments, the balloon catheter is part of a transcatheter heart valve delivery system and wherein positioning the balloon shoulder assembly within the main balloon of the balloon catheter includes creating a valve sitting pocket on the main balloon, in a space between the proximal balloon shoulder and the distal balloon shoulder, for a prosthetic heart valve and that reduces movement of the prosthetic heart valve during an implantation procedure with the transcatheter heart valve delivery system.

In another representative embodiment, a balloon catheter for an endovascular delivery system can include a proximal balloon shoulder mounted on an inner shaft of the balloon catheter, the proximal balloon shoulder comprising a compressible, hollow shell; a distal balloon shoulder mounted on the inner shaft, the distal balloon shoulder comprising a compressible, hollow shell; and an inflatable, main balloon that encloses the proximal balloon shoulder and the distal balloon shoulder.

In some embodiments, the proximal balloon shoulder and the distal balloon shoulder are blow molded together as one piece with a central connection portion extending therebetween.

In some embodiments, the proximal balloon shoulder and the distal balloon shoulder are each blow molded as a separate piece.

In some embodiments, the proximal balloon shoulder and the distal balloon shoulder are each fully enclosed and sealed around the inner shaft and inflatable.

In some embodiments, each of the proximal balloon shoulder and the distal balloon shoulder has an open, non-sealed end around the inner shaft and a wall thickness in a range of 0.001 inch to 0.010 inch that is adapted to support the main balloon.

In some embodiments, the proximal balloon shoulder and the distal balloon shoulder each include a collar portion and a shaft portion, the collar portion extending radially outward from the shaft portion.

In some embodiments, the collar portion has one of a funnel, conical, ellipsoid, or spheroid shape.

In some embodiments, the collar portion has a central portion arranged between tapered end portions, the central portion having one of a cylindrical, ring, or bulbous, curved shape.

In some embodiments, the main balloon is adapted to receive a prosthetic heat valve crimped thereon, between the proximal balloon shoulder and the distal balloon shoulder.

In some embodiments, the inner shaft extends distally beyond an outer shaft of the balloon catheter and through the main balloon.

In another representative embodiment, a balloon catheter for an endovascular delivery system can include a proximal balloon shoulder mounted on an outer shaft of the balloon catheter, the proximal balloon shoulder comprising a compressible, hollow shell; a distal balloon shoulder mounted on an inner shaft of the balloon catheter, the distal balloon shoulder comprising a compressible, hollow shell; and an inflatable, main balloon that encloses the proximal balloon shoulder and the distal balloon shoulder.

In some embodiments, the proximal balloon shoulder and the distal balloon shoulder are blow molded together as one piece with a central connection portion extending therebetween.

In some embodiments, the proximal balloon shoulder and the distal balloon shoulder are each blow molded as a separate piece and are spaced axially apart from one another.

In some embodiments, the proximal balloon shoulder is fully enclosed and sealed around the outer shaft and is inflatable and the distal balloon shoulder is fully enclosed and sealed around the inner shaft and is inflatable.

In some embodiments, the proximal balloon shoulder has an open, non-sealed end around the outer shaft and the distal balloon shoulder has an open, non-sealed end around the inner shaft.

In some embodiments, the proximal balloon shoulder and the distal balloon shoulder each include a collar portion and a shaft portion, the collar portion extending radially outward from the shaft portion.

In some embodiments, the collar portion has one of a funnel, conical, ellipsoid, or spheroid shape.

In some embodiments, the collar portion has a central portion arranged between tapered end portions, the central portion having one of a cylindrical, ring, or bulbous, curved shape.

In some embodiments, the main balloon is adapted to receive a prosthetic heat valve crimped thereon, between the proximal balloon shoulder and the distal balloon shoulder.

In some embodiments, the inner shaft extends distally beyond the outer shaft of the balloon catheter and through the main balloon.

In another representative embodiment, a balloon catheter for an endovascular delivery system can include a proximal balloon shoulder mounted on an outer shaft of the balloon catheter, the proximal balloon shoulder comprising a compressible, hollow shell; a distal balloon shoulder mounted on a nosecone of the balloon catheter, the distal balloon shoulder comprising a compressible, hollow shell; and an inflatable, main balloon that encloses the proximal balloon shoulder and the distal balloon shoulder.

In some embodiments, the proximal balloon shoulder and the distal balloon shoulder are blow molded together as one piece with a central connection portion extending therebetween.

In some embodiments, the proximal balloon shoulder and the distal balloon shoulder are each blow molded as a separate piece and are spaced axially apart from one another.

In some embodiments, the proximal balloon shoulder is fully enclosed and sealed around the outer shaft and is inflatable and the distal balloon shoulder is fully enclosed and sealed around the nosecone and is inflatable.

In some embodiments, the proximal balloon shoulder has an open, non-sealed end around the outer shaft and the distal balloon shoulder has an open, non-sealed end around the nosecone.

In some embodiments, the proximal balloon shoulder and the distal balloon shoulder each include a collar portion and a shaft portion, the collar portion extending radially outward from the shaft portion.

In some embodiments, the collar portion has one of a funnel, conical, ellipsoid, or spheroid shape.

In some embodiments, the collar portion has a central portion arranged between tapered end portions, the central portion having one of a cylindrical, ring, or bulbous, curved shape.

In some embodiments, the main balloon is adapted to receive a prosthetic heat valve crimped thereon, between the proximal balloon shoulder and the distal balloon shoulder.

In some embodiments, the inner shaft extends distally beyond the outer shaft of the balloon catheter and through the main balloon and the nosecone is coupled to a distal end of the inner shaft.

In another representative embodiment, a balloon catheter for an endovascular delivery system can include a proximal balloon shoulder mounted on an inner shaft of the balloon catheter, the proximal balloon shoulder comprising a compressible, hollow shell; a distal balloon shoulder mounted on a nosecone of the balloon catheter, the distal balloon shoulder comprising a compressible, hollow shell; and an inflatable, main balloon that encloses the proximal balloon shoulder and the distal balloon shoulder.

In some embodiments, the proximal balloon shoulder and the distal balloon shoulder are blow molded together as one piece with a central connection portion extending therebetween.

In some embodiments, the proximal balloon shoulder and the distal balloon shoulder are each blow molded as a separate piece and are spaced axially apart from one another.

In some embodiments, the proximal balloon shoulder is fully enclosed and sealed around the inner shaft and is inflatable and the distal balloon shoulder is fully enclosed and sealed around the nosecone and is inflatable.

In some embodiments, the proximal balloon shoulder has an open, non-sealed end around the inner shaft and the distal balloon shoulder has an open, non-sealed end around the nosecone.

In some embodiments, the proximal balloon shoulder and the distal balloon shoulder each include a collar portion and a shaft portion, the collar portion extending radially outward from the shaft portion.

In some embodiments, the collar portion has one of a funnel, conical, ellipsoid, or spheroid shape.

In some embodiments, the collar portion has a central portion arranged between tapered end portions, the central portion having one of a cylindrical, ring, or bulbous, curved shape.

In some embodiments, the main balloon is adapted to receive a prosthetic heat valve crimped thereon, between the proximal balloon shoulder and the distal balloon shoulder.

In some embodiments, the inner shaft extends distally beyond an outer shaft of the balloon catheter and through the main balloon and the nosecone is coupled to a distal end of the inner shaft.

The foregoing and other objects, features, and advantages of the invention will become more apparent from the following detailed description, which proceeds with reference to the accompanying figures.

### BRIEF DESCRIPTION OF THE DRAWINGS

FIG. 1 is a perspective view of a prosthetic heart valve, according to one embodiment.
FIG. 2A is a perspective view of a prosthetic heart valve, according to another embodiment.
FIG. 2B is a perspective view of the prosthetic valve of FIG. 2A with the components on the outside of the frame shown in transparent lines for purpose of illustration.
FIG. 3 is a perspective view of a delivery device for a prosthetic heart valve, according to an embodiment.
FIG. 4 is a cross-sectional view of an embodiment of a distal end portion of the delivery device of FIG. 3.
FIG. 5 is a side view of an assembly including a prosthetic valve crimped on a balloon mounted on a distal end portion of a balloon catheter, according to one embodiment.
FIG. 6 is a side view of a blow molded balloon shoulder assembly for a balloon catheter, according to a first embodiment.
FIG. 7 is a side view of a blow molded balloon shoulder assembly for a balloon catheter, according to a second embodiment.
FIG. 8 is a side view of balloon shoulders of a balloon shoulder assembly for a balloon catheter, according to one embodiment.
FIG. 9 is a side view of balloon shoulders of a balloon shoulder assembly for a balloon catheter, according to another embodiment.
FIG. 10 is a side view of balloon shoulders of a balloon shoulder assembly for a balloon catheter, according to yet another embodiment.
FIG. 11 is a side view of an inflatable balloon shoulder assembly in a non-inflated state, according to one embodiment.
FIG. 12 is a side view of the inflatable balloon shoulder assembly of FIG. 11 in an inflated state.
FIG. 13 is a side view of a non-inflatable balloon shoulder assembly, according to one embodiment.
FIG. 14 is a flow chart of a method for manufacturing a balloon catheter including a blow molded balloon shoulder assembly.
FIGS. 15A-15C show various ways of mounting a balloon shoulder assembly on a balloon catheter.

### DETAILED DESCRIPTION

Described herein are examples of a blow molded balloon shoulder assembly for a transcatheter delivery system (e.g., a transcatheter heart valve delivery system) and methods for manufacturing blow molded balloon shoulder assemblies and balloon catheters including the blow molded balloon shoulder assemblies. The balloon shoulder assembly may include a proximal balloon shoulder including a collar portion (e.g., flared end) and a shaft portion (e.g., shaft end) and a distal balloon shoulder including a collar portion and a shaft portion. Each of the proximal balloon shoulder and the distal balloon shoulder may be hollow and comprise a compressible, blow molded material. The balloon shoulder assembly may be inserted into an inflatable, main balloon of a balloon catheter of the delivery system. In some examples, the collar portions of the proximal and distal balloon shoulders may be spaced apart from one another inside the main balloon to form a valve (or other implantable device) retaining portion on the balloon, where the valve retaining portion is adapted to receive a crimped prosthetic heart valve, in some embodiments. In other embodiments, the valve retaining portion may be a device retaining portion adapted to receive another type of implantable medical device, such as a stent. The balloon shoulder assembly may be formed via blow molding, thereby generating hollow, compressible balloon shoulders. For example, outer walls of the balloon shoulders, formed via blow molding, may compress (or stretch) under pressure and return back to their uncompressed or unstretched state upon removal of the applied pressure. Blow molding the balloon shoulders creates less bulky and more resilient (e.g., flexible) balloon shoulders, with similar structural strength, as compared to traditional, injection molded balloon shoulders. As a result, the blow molded balloon shoulder assembly may adequately support the main balloon of the balloon catheter, while being able to compress during insertion into the balloon and during a retrieval process where the balloon catheter is retracted from the implantation site and the patient's body. Thus, balloon catheters including the blow molded balloon shoulder assemblies may be easier to manufacture and easier to maneuver through an internal lumen of a patient, while also reducing degradation of components of the delivery system.

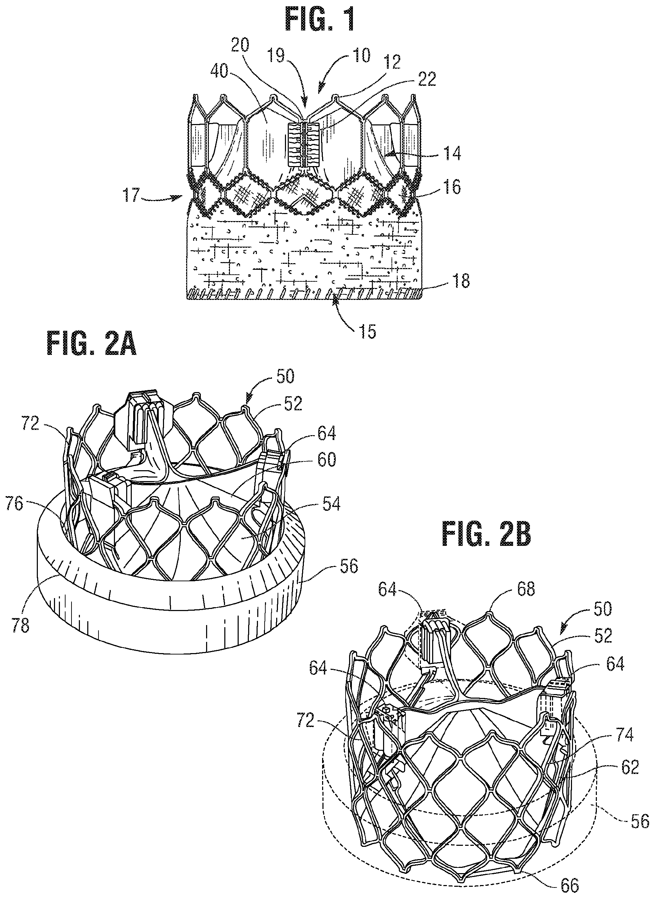

In some embodiments, the balloon catheter is adapted to deliver a prosthetic heart valve crimped onto the valve retaining portion of the main balloon, between the distal and proximal balloon shoulders. FIG. 1 shows a prosthetic heart valve 10, according to one embodiment. The illustrated prosthetic valve is adapted to be implanted in the native aortic annulus, although in other embodiments it can be adapted to be implanted in the other native annuluses of the heart (e.g., the pulmonary, mitral, and tricuspid valves). The prosthetic valve can also be adapted to be implanted in other tubular organs or passageways in the body. The prosthetic valve 10 can have four main components: a stent or frame 12, a valvular structure 14, an inner skirt 16, and a perivalvular outer sealing member or outer skirt 18. The prosthetic valve 10 can have an inflow end portion 15, an intermediate portion 17, and an outflow end portion 19.

The valvular structure 14 can comprise three leaflets 40, collectively forming a leaflet structure, which can be arranged to collapse in a tricuspid arrangement, although in other embodiments there can be greater or fewer number of leaflets (e.g., one or more leaflets 40). The leaflets 40 can be secured to one another at their adjacent sides to form commissures 22 of the leaflet structure 14. The lower edge of valvular structure 14 can have an undulating, curved scalloped shape and can be secured to the inner skirt 16 by sutures (not shown). In some embodiments, the leaflets 40 can be formed of pericardial tissue (e.g., bovine pericardial tissue), biocompatible synthetic materials, or various other suitable natural or synthetic materials as known in the art and described in U.S. Patent No. 6,730,118, which

The frame 12 can be formed with a plurality of circumferentially spaced slots, or commissure windows 20 that are adapted to mount the commissures 22 of the valvular structure 14 to the frame. The frame 12 can be made of any of various suitable plastically-expandable materials (e.g., stainless steel, etc.) or self-expanding materials (e.g., nickel titanium alloy (NiTi), such as nitinol), as known in the art. When constructed of a plastically-expandable material, the frame 12 (and thus the prosthetic valve 10) can be crimped to a radially collapsed configuration on a delivery catheter and then expanded inside a patient by an inflatable balloon or equivalent expansion mechanism. When constructed of a self-expandable material, the frame 12 (and thus the prosthetic valve 10) can be crimped to a radially collapsed configuration and restrained in the collapsed configuration by insertion into a sheath or equivalent mechanism of a delivery catheter. Once inside the body, the prosthetic valve can be advanced from the delivery sheath, which allows the prosthetic valve to expand to its functional size.

Suitable plastically-expandable materials that can be used to form the frame 12 include, without limitation, stainless steel, a biocompatible, high-strength alloys (e.g., a cobalt-chromium or a nickel-cobalt-chromium alloys), polymers, or combinations thereof. In particular embodiments, frame 12 is made of a nickel-cobalt-chromium-molybdenum alloy, such as MP35N° alloy (SPS Technologies, Jenkintown, Pennsylvania), which is equivalent to UNS R30035 alloy (covered by ASTM F562-02). MP35N° alloy/UNS R30035 alloy comprises 35% nickel, 35% cobalt, 20% chromium, and 10% molybdenum, by weight. Additional details regarding the prosthetic valve 10 and its various components are described in WIPO Patent Application Publication No. WO 2018/222799,

FIG. 2A is a perspective view of a prosthetic heart valve 50, according to another embodiment. The valve 50 can have three main components: a stent or frame, 52, a valvular structure 54, and a sealing member 56. FIG. 2B is a perspective view of the prosthetic valve 50 with the components on the outside of the frame 52 (including the sealing member 56) shown in transparent lines for purposes of illustration.

Like the valvular structure 14 of FIG. 1, the valvular structure 54 can comprise three leaflets 60, collectively forming a leaflet structure, which can be arranged to collapse in a tricuspid arrangement. Each leaflet 60 can be coupled to the frame 52 along its inflow edge 62 (the lower edge in the figures; also referred to as "cusp edges") and at commissures 64 of the valvular structure 54 where adjacent portions of two leaflets are connected to each other. A reinforcing element (not shown), such as a fabric strip, can be connected directly to the cusp edges of the leaflets and to the struts of the frame to couple the cusp edges of the leaflets to the frame.

Similar to the frame 12 of FIG. 1, the frame 52 can be made of any of various suitable plastically-expandable materials or self-expanding materials, as known in the art and described above. The frame 52 in the illustrated embodiment comprises a plurality of circumferentially extending rows of angled struts 72 defining rows of cells, or openings, 74 of the frame. The frame 52 can have a cylindrical or substantially cylindrical shape having a constant diameter from an inflow end 66 to an outflow end 68 of the frame as shown, or the frame can vary in diameter along the height of the frame, as disclosed in U.S. Patent Publication No. 2012/0239142,

The sealing member 56 in the illustrated embodiment is mounted on the outside of the frame 52 and functions to create a seal against the surrounding tissue (e.g., the native leaflets and/or native annulus) to prevent or at least minimize paravalvular leakage. The sealing member 56 can comprise an inner layer 76 (which can be in contact with the outer surface of the frame 52) and an outer layer 78. The sealing member 56 can be connected to the frame 52 using suitable techniques or mechanisms. For example, the sealing member 56 can be sutured to the frame 52 via sutures that can extend around the struts 72 and through the inner layer 76. In alternative embodiments, the inner layer 76 can be mounted on the inner surface of the frame 52, while the outer layer 78 is on the outside of the frame 52.

The outer layer 78 can be configured or shaped to extend radially outward from the inner layer 76 and the frame 52 when the prosthetic valve 50 is deployed. When the prosthetic valve is fully expanded outside of a patient's body, the outer layer 78 can expand away from the inner layer 76 to create a space between the two layers. Thus, when implanted inside the body, this allows the outer layer 78 to expand into contact with the surrounding tissue.

Additional details regarding the prosthetic valve 50 and its various components are described in U.S. Patent Publication No. 2018/0028310,

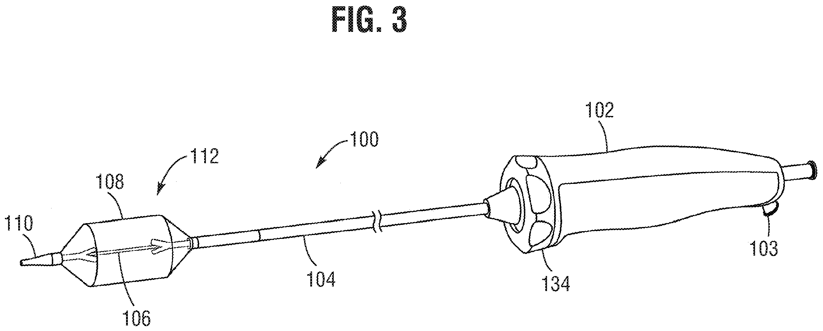

FIGS. 3-5 show various embodiments and components of a delivery system, such as a transcatheter delivery system, including a delivery device (which, in one embodiment, may be a balloon catheter). FIG. 3 shows a delivery device (e.g., apparatus) 100, according to an embodiment, that can be used to implant an expandable prosthetic heart valve (e.g., heart valve 10 or 50), or another type of expandable prosthetic medical device (such as a stent). In some embodiments, the delivery device 100 is specifically adapted for use in introducing a prosthetic valve into a heart.

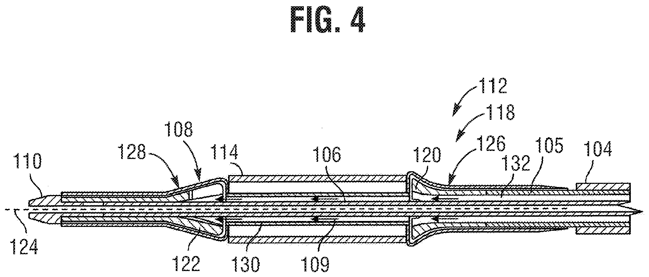

Referring to FIG. 3, the delivery device 100 in the illustrated embodiment is a balloon catheter comprising a handle 102, a steerable, outer shaft 104 extending from the handle 102, an intermediate shaft 105 (see FIG. 4) extending from the handle 102 coaxially through the steerable outer shaft 104, and an inner shaft 106 extending from the handle 102 coaxially through the intermediate shaft 105 and the steerable outer shaft 104, an inflatable balloon 108 extending from a distal end of the intermediate shaft 105, and a nosecone 110 arranged at a distal end of the delivery device 100. A distal end portion 112 of the delivery device 100 includes the balloon 108, the nosecone 110, and a balloon shoulder assembly. A prosthetic medical device, such as a prosthetic heart valve may be mounted on a valve retaining portion of the balloon 108, as described further below with reference to FIG. 4. As described further below, the balloon shoulder assembly is configured to maintain the prosthetic heart valve or other medical device at a fixed position on the balloon 108 during delivery through the patient's vasculature.

The inner shaft 106 can define an inner lumen that is configured to receive a guidewire therein. For example, during delivery of the implantable medical device (e.g., prosthetic heart valve) to the target implantation site with the delivery device 100, the delivery device 100 can be advanced over the guidewire to the target implantation site.

The handle 102 can include a steering mechanism configured to adjust the curvature of the distal end portion of the delivery device. In the illustrated embodiment, for example, the handle 102 includes an adjustment member, such as the illustrated rotatable knob 134, which in turn is operatively coupled to the proximal end portion of a pull wire (not shown). The pull wire extends distally from the handle 102 through the outer shaft 104 and has a distal end portion affixed to the outer shaft at or near the distal end of the outer shaft 104. Rotating the knob 134 is effective to increase or decrease the tension in the pull wire, thereby adjusting the curvature of the distal end portion of the delivery device.

FIG. 4 shows an embodiment of the distal end portion 112 of the delivery device 100. As shown in FIG. 4, the delivery device 100 is configured to mount a prosthetic valve (e.g., prosthetic heart valve) 114 in a crimped state over the balloon 108 for insertion of the delivery device 100 and prosthetic valve 114 into a patient's vasculature.

As shown in FIG. 4, at a proximal end of the distal end portion 112, the inner shaft 106 extends distally beyond the steerable outer shaft 104 and the intermediate shaft 105 and through the balloon 108. The balloon 108 can be supported on a balloon shoulder assembly 118. The balloon shoulder assembly 118 includes a proximal shoulder 120 connected to a distal end of the intermediate shaft 105 and a distal shoulder 122 mounted on the inner shaft 106. The balloon 108 includes a proximal end portion 126 surrounding and/or folded over the proximal shoulder 120 and a distal end portion 128 surrounding and/or folded over the distal shoulder 122. In some embodiments, the proximal end portion 126 of the balloon 108 may be secured to the outer surface of the intermediate shaft 105. In some embodiments, the distal end portion 128 of the balloon 108 may be secured to the outer surface of the nosecone 110, which can be mounted on or coupled to the inner shaft 106.

In the illustrated embodiment, the nosecone 110 and the distal shoulder 122 can be a one-piece or unitary component, that is, the nosecone 110 is a distal portion of the unitary component and the distal shoulder 122 is a proximal portion of the unitary component. In other embodiments, the nosecone 110 and the distal shoulder 122 can be separate components, and each can be mounted on the inner shaft 106 next to each other or at axially spaced locations.

The proximal shoulder 120 and the distal shoulder 122 are spaced apart from one another, in an axial direction relative to a central longitudinal axis 124 of the delivery device 100. As a result, the balloon 108 defines a valve retaining portion 130 in the space that separates the proximal shoulder 120 and the distal shoulder 122 (e.g., between flared ends of the proximal shoulder 120 and the distal shoulder 122). As shown in FIG. 4, the prosthetic valve 114 can be crimped onto the valve retaining portion 130 (also may be referred to as a valve sitting pocket) of the balloon 108, between the proximal shoulder 120 and the distal shoulder 122, thereby preventing or reducing axial movement of the prosthetic valve 114 relative to the balloon 108 during insertion of the delivery device 100 into the patient and delivery of the prosthetic valve 114 to the target implantation site.

The outer diameter of the inner shaft 106 can be sized such that an annular space 132 is defined between the inner shaft 106 and the intermediate shaft 105 along the entire length of the intermediate shaft 105. The annular space 132 may be fluidly coupled to one or more fluid passageways of the delivery device 100 which can be fluidly connectable to a fluid source (e.g., a syringe) that can inject an inflation fluid (e.g., saline) into the delivery device. In this way, fluid from the fluid source can flow through the one or more fluid passageways, through the annular space 132, and into the balloon 108 to inflate the balloon 108 and expand and deploy the prosthetic valve 114. For example, the handle 102 can have a fluid port 103 (see FIG. 3) configured to be coupled to the fluid source. In use, inflation fluid from the fluid source can be injected into the fluid port 103, through one or more fluid passageways in the handle 102, through the annular space 132, and into the balloon 108.

FIG. 4 illustrates the flow of fluid (indicated by arrows 109) through the annular space 132 and through passages in the proximal shoulder 120 and distal shoulder 122. The fluid can then flow into the proximal and distal end portions 126, 128 of the balloon 108 to expand the valve 114. Further details of the balloon shoulder assembly, the steering mechanism, and other components of the delivery device are disclosed in U.S. Publication Nos. 2007/0005131, 2009/0281619, 2013/0030519, and 2017/0065415,

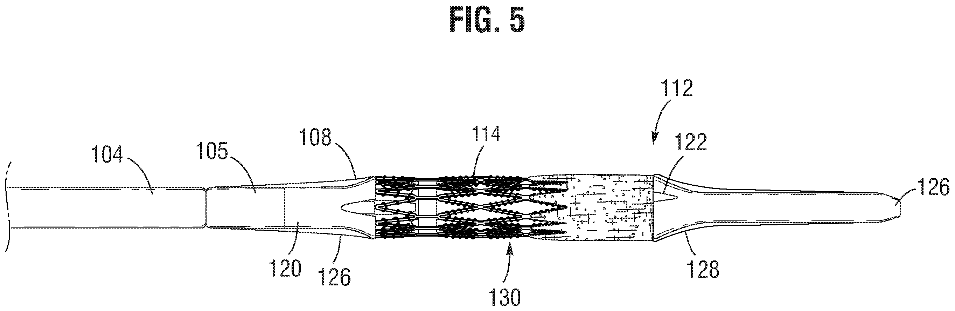

FIG. 5 shows a side view of an exterior of the distal end portion 112 of the delivery device 100, including the prosthetic valve 114 crimped on the balloon 108 mounted on the distal end portion 112 of the balloon catheter. As explained above, the balloon shoulder assembly including the proximal shoulder 120 and distal shoulder 122 supports the balloon 108 thereon.

As shown in FIG. 5, the balloon 108 includes the proximal end portion 126 surrounding and/or folded over the proximal shoulder 120, the distal end portion 128 surrounding and/or folded over the distal shoulder 122, and the valve retaining portion 130 located between the proximal end portion 126 and the distal end portion 128. As shown in FIG. 5, the prosthetic valve 114 is crimped to the balloon catheter, on and around the valve retaining portion 130.

Traditionally, injection molded balloon shoulder assemblies, such as the balloon shoulders shown in FIG. 5, may be thick parts that are relatively hard (e.g., comprise a solid, hard plastic material). Overall, injection molded balloon shoulders may be bulky, and thus, may have a relatively long length to accommodate the increased mass. As a result of their hard, thick, and stiff nature, injection molded balloon shoulders may be difficult to insert into the relatively narrow legs of the balloon of the balloon catheter, during manufacturing of the balloon catheter. Further, these characteristics may also create gaps between the injection molded balloon shoulders and a valve (or other implantable device) crimped around the balloon, between the proximal and distal balloon shoulders, which may result in damage to a sheath or alternate medical instrument through which the balloon catheter is inserted while navigating curves within a patient's vasculature. Further still, due to the bulky and hard nature of the injection molded shoulders, higher retrieving forces may be experienced by a user while attempting to retrieve (e.g., remove) the balloon catheter through the sheath following valve deployment at the target site in the patient.

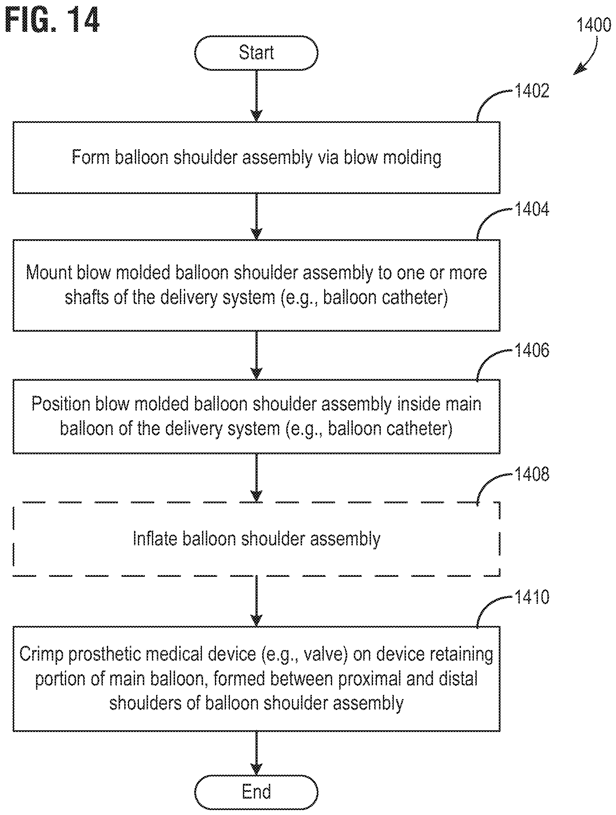

As explained further below with reference to FIG. 14, balloon shoulders and/or balloon shoulder assemblies for a balloon catheter may instead be formed via blow molding (as opposed to injection molding). Blow molding creates plastic, hollow parts with decreased mass (e.g., bulk), similar structural strength, decreased stiffness, decreased hardness, and increased compressibility (e.g., resilience) relative to injection molded parts. Further, blow molding may make it possible to change a design, such as the size and shape of a part, more quickly and cheaply than injection molding (e.g., due to the expensive nature of the molds required for injection molding). As a result, blow molded balloon shoulders may be more resilient, allowing them to compress and pop back into an expanded shape, while still providing ample structural support to the main balloon, thereby making them easier to insert within the main balloon of a balloon catheter and reducing degradation to a sheath during maneuvering the balloon catheter through the sheath during an implantation procedure. Further, a balloon catheter including the blow molded balloon shoulders and/or balloon shoulder assembly may be easier to retrieve (e.g., with reduced force or resistance) through the sheath and/or patient's vasculature after implantation of the prosthetic medical device. Additionally, as explained further below, because blow molded parts are hollow and compressible, blow molded balloon shoulders may be configured to be inflated and deflated (e.g., function as additional balloons).

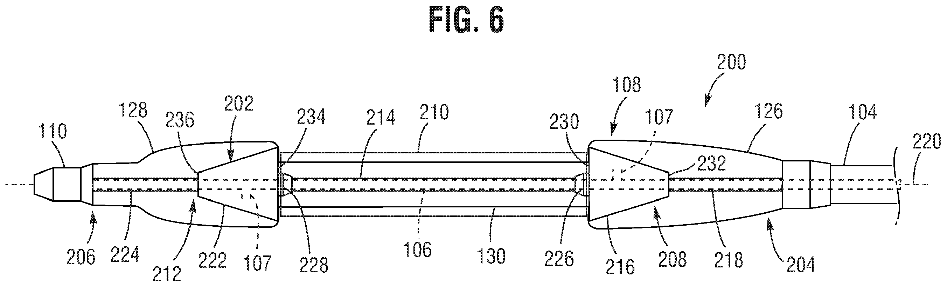

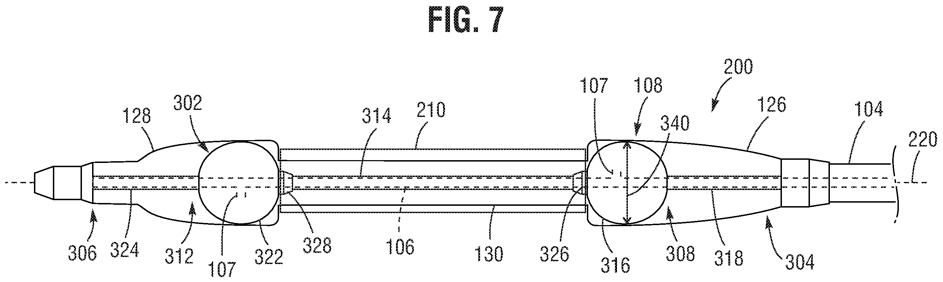

FIGS. 6 and 7 show embodiments of a blow molded balloon shoulder assembly included in an exemplary balloon catheter 200 (which can also be referred to as a delivery apparatus or delivery device). The balloon catheter 200 may be similar to the delivery device (e.g., balloon catheter) 100 depicted in FIGS. 3-5, as explained above. As such, similar components to those of the delivery device (e.g., balloon catheter) 100 of FIGS. 3-5 have been numbered the same in the balloon catheter 200 of FIGS. 6-7.

Turning first to FIG. 6, a first embodiment of a blow molded balloon shoulder assembly 202 for the balloon catheter 200 is shown. As introduced above with reference to FIG. 5, the balloon catheter 200 includes an inflatable main balloon 108 (shown in a deflated state in FIGS. 4-7), an outer shaft 104, and an inner shaft 106 extending through the outer shaft 104 and the main balloon 108. As explained above with reference to FIGS. 4-5, the balloon 108 includes a proximal end portion 126, a distal end portion 128, and a valve retaining portion 130 arranged between the proximal end portion 126 and the distal portion 128. In the illustrated embodiment, the distal end portion 128 of the balloon can be connected to a nosecone 110 and the proximal end portion 126 of the balloon can be connected to the outer shaft 104. A prosthetic medical device (e.g., a prosthetic heart valve) 210 is crimped on the deflated balloon 108, and is depicted in FIGS. 6-7 as a box for illustration purposes. In some embodiments, the prosthetic medical device 210 may be one of the prosthetic heart valve 10 shown in FIG. 1, the prosthetic heart valve 50 shown in FIGS. 2A-2B, or the prosthetic heart valve 114 shown in FIGS. 4-5. In alternate embodiments, the prosthetic medical device 210 may be another type of implantable medical device, such as a stent or graft.

The balloon shoulder assembly 202 may be mounted on the inner shaft 106. In some embodiments, as shown in FIG. 6, the balloon shoulder assembly 202 is formed as one piece that extends from a proximal end 204 to a distal end 206 of the balloon shoulder assembly 202 and surrounds an entire outer circumference of the inner shaft 106. In alternate embodiments, as explained further below, the balloon shoulder assembly 202 may not be formed as one piece and portions of the inner shaft 106 may not be surrounded by the balloon shoulder assembly 202 (e.g., a central portion in the region of valve retaining portion 130).

As shown in FIG. 6, the balloon shoulder assembly 202 includes a proximal balloon shoulder 208, a distal balloon shoulder 212, and a central connecting portion 214 that connects the proximal balloon shoulder 208 and the distal balloon shoulder 212. In some embodiments, the central connecting portion 214 is formed as one piece with the proximal balloon shoulder 208 and the distal balloon shoulder 212. In other embodiments, the proximal balloon shoulder 208, the distal balloon shoulder 212, and the central connecting portion 214 are formed (e.g., blow molded) as separate pieces and then coupled together (e.g., via an adhesive or alternate bonding technique).

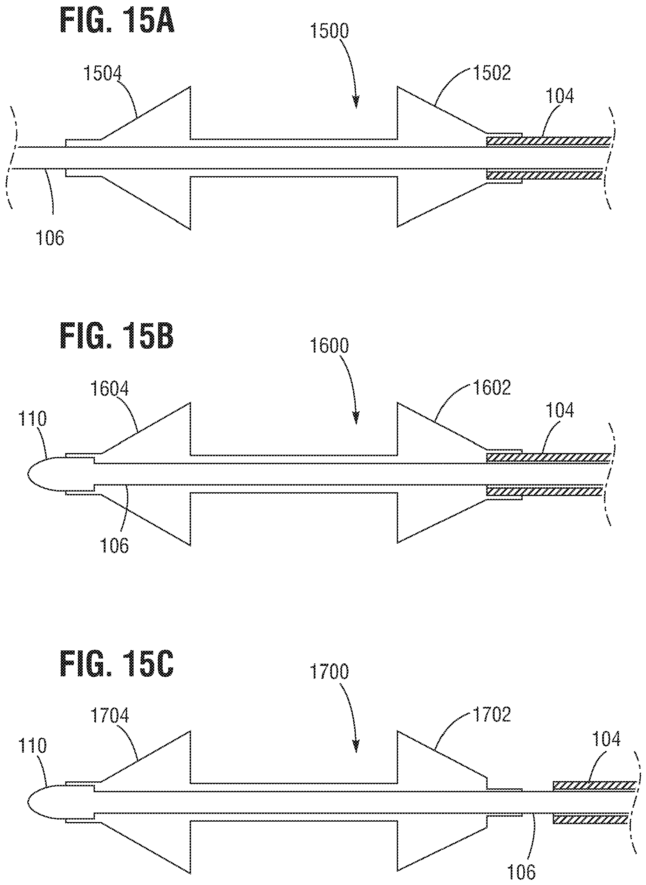

As noted above, in some embodiments, the entire balloon shoulder assembly 202 can be mounted on the inner shaft 106. In other embodiments, such as those discussed further below with reference to FIGS. 15A-15C, the proximal balloon shoulder 208 can be mounted on or to the inner shaft 106 or the outer shaft 104, and the distal balloon shoulder 212 can be mounted on or to the inner shaft 106 or a nosecone 110.

The proximal balloon shoulder 208 includes a proximal collar portion (e.g., member or flared end) 216 and a proximal shaft portion (e.g., member or shaft end) 218. Specifically, the proximal collar portion 216 extends radially outward, relative to a central axis 220 of the balloon catheter 200 and the balloon shoulder assembly 202, from the proximal shaft portion 218. The proximal shaft portion 218 has a smaller diameter than a remainder of the proximal balloon shoulder 208 (e.g., the proximal collar portion 216) and this smaller diameter of the proximal shaft portion 218 may be similar to (or slightly bigger than, in some embodiments) a diameter of the inner shaft 106. Additionally, the proximal collar portion 216 is arranged at a first, more distal, end of the proximal balloon shoulder 208 (adjacent to the central connecting portion 214) and the proximal shaft portion 218 is arranged at an opposite, second, more proximal, end of the proximal balloon shoulder 208. In some embodiments, a length of the proximal shaft portion 218 may be longer than a length of the proximal collar portion 216. In some embodiments, the proximal shaft portion 218 may extend through at least a portion of an interior of the proximal collar portion 216.

Similarly, the distal balloon shoulder 212 includes a distal collar portion (e.g., member or flared end) 222 and a distal shaft portion (e.g., member or shaft end) 224. Specifically, the distal collar portion 222 extends radially outward, relative to the central axis 220, from the distal shaft portion 224. The distal shaft portion 224 has a smaller diameter than a remainder of the distal balloon shoulder 212 (e.g., the distal collar portion 222) and this smaller diameter of the distal shaft portion 224 may be similar to (or slightly bigger than, in some embodiments) the diameter of the inner shaft 106. Additionally, the distal collar portion 222 is arranged at a first, more proximal, end of the distal balloon shoulder 212 (adjacent to the central connecting portion 214) and the distal shaft portion 224 is arranged at an opposite, second, more distal, end of the distal balloon shoulder 212. In some embodiments, a length of the distal shaft portion 224 may be longer than a length of the distal collar portion 222. In some embodiments, the distal shaft portion 224 may extend through at least a portion of an interior of the distal collar portion 222.

In some embodiments, as shown in FIG. 6, the central connecting portion 214 is an elongate, hollow shaft that extends between and connects the distal collar portion 222 and the proximal collar portion 216.

In some embodiments, as shown in FIG. 6, the balloon shoulder assembly 202 may further include transition portions which extend between and connect each collar portion to the central connecting portion 214. In some embodiments, the transition portions may also be formed as one piece with a remainder of the balloon shoulder assembly 202. In some embodiments, the transition portions may be part of the central connecting portion 214 or part of a corresponding collar portion. For example, as shown in FIG. 6, the balloon shoulder assembly 202 includes a proximal transition portion 226 extending between, in the axial direction, and connecting the proximal collar portion 216 and the central connecting portion 214 and a distal transition portion 228 extending between, in the axial direction, and connecting the distal collar portion 222 and the central connecting portion 214. The transition portions 226 and 228 may each narrow in diameter from a corresponding collar portion to the central connecting portion 214. In alternate embodiments, the balloon shoulder assembly 202 may not include transition portions or the transition portions may have a different shape than depicted in FIG. 6 (e.g., stepped).

As shown in FIG. 6, each of the proximal collar portion 216 and the distal collar portion 222 have a funnel shape (e.g., a conical shape with a wider end and a narrower end). For example, the proximal collar portion 216 has a wider end 230 that flares radially outward from a narrower end 232 of the proximal collar portion 216. The wider end 230 is connected to the central connecting portion 214 and the narrower end 232 is connected to the proximal shaft portion 218. Similarly, the distal collar portion 222 has a wider end 234 that flares radially outward from a narrower end 236 of the distal collar portion 222. The wider end 234 is connected to the central connecting portion 214 and the narrower end 236 is connected to the distal shaft portion 224.

As introduced above, the balloon shoulder assembly 202 is formed via blow molding. As such, the balloon shoulder assembly 202, including the proximal balloon shoulder 208 and the distal balloon shoulder 212, is hollow and comprises a compressible, blow molded material. The blow molded material may be a polymer, in some embodiments. In some embodiments, the blow molded material can be any of various polymers, such as polyethylene, polypropylene, polyurethane, nylon, PET, PBT, or the like. As explained above and further below, by having balloon shoulders that are hollow and compressible, due to being blow molded, the balloon shoulders may provide structural support to the main balloon 108 while making them easier to insert within the main balloon 108 during assembly and maneuver through a patient's vasculature.

In some embodiments, as shown in FIG. 6, the proximal balloon shoulder 208, the distal balloon shoulder 212, the central connecting portion 214, and the transition portions 226 and 228 (if included) may be molded together and formed as one piece via blow molding. In alternate embodiments, the proximal balloon shoulder 208 and the distal balloon shoulder 212 may be blow molded separately, as two different parts (and not formed as one piece). In some of these embodiments, the balloon shoulder assembly may not include the central connecting portion and instead the separately blow molded proximal and distal balloon shoulders may be spaced apart from one another. Thus, in some embodiments, the balloon shoulder assembly 202 may include the proximal balloon shoulder 208 and distal balloon shoulder 212, as shown in FIG. 6, but without the central connecting portion 214. In other of these embodiments, the separately molded proximal balloon shoulder 208 and distal balloon shoulder 212 may be coupled to the central connecting portion, after blow molding and before insertion into a main balloon of the balloon catheter.

Referring again to FIG. 6, in some embodiments, the proximal shaft portion 218 and the distal shaft portion 224 can be fixed to the inner shaft 106 using various techniques and mechanisms, such as by welding or an adhesive.

In some embodiments, the balloon shoulder assembly 202 may be fully enclosed and inflatable (e.g., capable of being inflated from a deflated state). As used herein, "fully enclosed" may refer to an enclosed or sealed structure, without holes or openings, that can retain pressure (e.g., fluid pressure). For example, the ends of each of the proximal shaft portion 218 and the distal shaft portion 224 not coupled to the corresponding collar portions, as well as both ends of the collar portions, may be closed or sealed to the respective shafts on which they are mounted. For example, each of the proximal shaft portion 218 and the distal shaft portion 224 can form a fluid tight seal against the outer surface of the inner shaft 106. In this way, the balloon shoulder assembly 202 may be completely enclosed and able to contain an inflation fluid introduced into the balloon shoulder assembly 202. As such, the balloon shoulder assembly 202, or portions of the balloon shoulder assembly (such as the proximal balloon shoulder 208 and distal balloon shoulder 212) may be a balloon (or balloons), in addition to the main balloon 108.

For example, in some embodiments, one or more shafts routed through an interior of the balloon catheter 200 may fluidly couple an external fluid source to the balloon shoulder assembly 202. As shown in FIG. 6, in some embodiments, the inner shaft 106 may have one or more side openings 107 in a region of the balloon shoulder assembly 202. Inflation fluid may then be provided from a fluid source (e.g., a syringe containing the inflation fluid) to a fluid port in the handle of the delivery apparatus, which fluid port is fluidly connected to a fluid passage formed within the inner shaft 106, which in turn is in fluid communication with the balloon shoulder assembly 202 via the one or more side openings 107. As such, inflation fluid may be provided to the one or more balloons of the balloon shoulder assembly 202 to inflate the balloon shoulder assembly 202.

In some embodiments, the inner surface of the central connecting portion 214 can be spaced radially outward from the outer surface of the inner shaft 106 to define an annular space through which the inflation fluid can flow from the proximal balloon shoulder 208 to the distal balloon shoulder 212. Thus, the inflation fluid can flow outwardly from the side opening 107 into the proximal balloon shoulder 208, through the annular space and into the distal balloon shoulder 212.

The side openings 107 shown in FIG. 6 (and FIG. 7) are for illustrative purposes and, in alternate embodiments, there may be more or less side openings 107 than those shown in FIGS. 6-7. Further, in some embodiments, the side openings 107 may be located in additional or alternate positions along the inner shaft 106 than those shown in FIGS. 6-7. Further, the side openings 107 may extend into an interior of the balloon shoulder assembly or be flush with an outer surface of the inner shaft 106.

In some embodiments, the balloon shoulder assembly 202 may be inflated during assembly of the balloon catheter 200, after inserting the deflated balloon shoulder assembly 202 into the main balloon 108. While the balloon shoulder assembly 202 is inflated, the main balloon 108 may be pleated and folded around the balloon shoulder assembly 202 and optionally, a protective cover or case can be placed around the main balloon for shipping and storage until use by a health care provider, as disclosed in U.S. Publication No. 2017/0065415. A prosthetic valve (e.g., valve 10 or 50) can be stored in a container or jar containing a hydrating fluid. At the point of use, the user can remove the prosthetic valve from its container, place the prosthetic valve around the main balloon and crimp the prosthetic valve onto the main balloon between the proximal and distal balloon shoulders 208 and 212, respectively.

In alternative embodiments, the prosthetic valve can have dry or substantially dry leaflets that can be stored without a hydrating fluid, such as disclosed in U.S. Patent Nos. 8,007,992 and 8,357,387. In such cases, the prosthetic valve can be crimped onto the main balloon during the assembly process and the assembly comprising the delivery apparatus and the crimped prosthetic valve can be placed in sterile package for shipping and storage until use by a health care provider.

In alternative embodiments, the balloon shoulder assembly 202 may be inflated at the point of use, such as prior to crimping the prosthetic valve on the main balloon.

Further, in some embodiments where the proximal balloon shoulder and distal balloon shoulder are molded separately, as separate pieces, they each may be additional balloons, in addition to the main balloon 108.

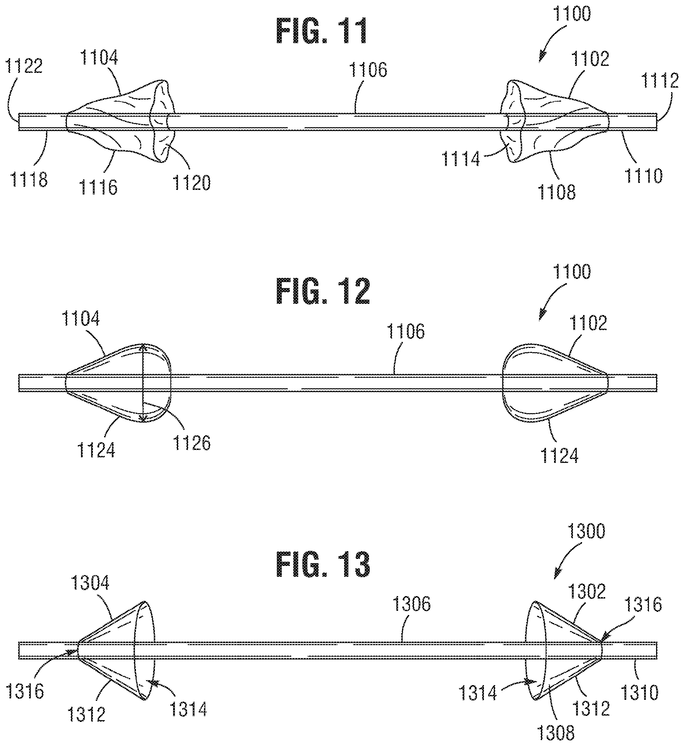

In alternate embodiments, the balloon shoulder assembly 202 may not be fully enclosed, and thus may not be able to contain pressure and be inflated (e.g., non-inflatable). Embodiments of blow molded balloon shoulders that are inflatable and embodiments of blow molded balloon shoulders that are non-inflatable are shown in FIGS. 11-13, as described further below.

FIG. 7 shows a second embodiment of a blow molded balloon shoulder assembly 302 for the balloon catheter 200. Similar to the balloon shoulder assembly 202 of FIG. 6, the balloon shoulder assembly 302 may be mounted on the inner shaft 106 of the balloon catheter 200. In some embodiments, as shown in FIG. 7, the balloon shoulder assembly 302 is formed as one piece that extends from a proximal end 304 to a distal end 306 of the balloon shoulder assembly 302 and surrounds an entire outer circumference of the inner shaft 106. Thus, the inner shaft 106 is not visible in FIG. 7. In alternate embodiments, as explained further below, the balloon shoulder assembly 302 may not be formed as one piece and portions of the inner shaft 106 may not be surrounded by the balloon shoulder assembly 302 (e.g., a central portion in the region of valve retaining portion 130).

As shown in FIG. 7, the balloon shoulder assembly 302 includes a proximal balloon shoulder 308, a distal balloon shoulder 312, and a central connecting portion 314 that connects the proximal balloon shoulder 308 and the distal balloon shoulder 312. In some embodiments, the central connecting portion 314 is formed as one piece with the proximal balloon shoulder 308 and the distal balloon shoulder 312. In other embodiments, the proximal balloon shoulder 308, the distal balloon shoulder 312, and the central connecting portion 314 are formed (e.g., blow molded) as separate pieces and then coupled together (e.g., via an adhesive or alternate bonding technique).

The proximal balloon shoulder 308 includes a proximal collar portion (e.g., member) 316 and a proximal shaft portion (e.g., member) 318. Specifically, the proximal collar portion 316 extends radially outward, relative to the central axis 220 of the balloon catheter 200 and the balloon shoulder assembly 302, from the proximal shaft portion 318. The proximal shaft portion 318 has a smaller diameter than a remainder of the proximal balloon shoulder 308 (e.g., the outer diameter of the proximal collar portion 316) and this smaller diameter of the proximal shaft portion 318 may be similar to (or slightly bigger than, in some embodiments) a diameter of the inner shaft 106. Additionally, the proximal collar portion 316 is arranged at a first, more distal, end of the proximal balloon shoulder 308 (adjacent to the central connecting portion 314) and the proximal shaft portion 318 is arranged at an opposite, second, more proximal, end of the proximal balloon shoulder 308. In some embodiments, a length of the proximal shaft portion 318 may be longer than a length of the proximal collar portion 316, in an axial direction relative to the central axis 220. In some embodiments, the proximal shaft portion 318 may extend through at least a portion of an interior of the proximal collar portion 316.

Similarly, the distal balloon shoulder 312 includes a distal collar portion (e.g., member) 322 and a distal shaft portion (e.g., member) 324. Specifically, the distal collar portion 322 extends radially outward, relative to the central axis 320, from the distal shaft portion 324. The distal shaft portion 324 has a smaller diameter than a remainder of the distal balloon shoulder 312 (e.g., the outer diameter of the distal collar portion 322) and this smaller diameter of the distal shaft portion 324 may be similar to (or slightly bigger than, in some embodiments) the diameter of the inner shaft 106. Additionally, the distal collar portion 322 is arranged at a first, more proximal, end of the distal balloon shoulder 312 (adjacent to the central connecting portion 314) and the distal shaft portion 324 is arranged at an opposite, second, more distal, end of the distal balloon shoulder 312. In some embodiments, a length of the distal shaft portion 324 (in the axial direction) may be longer than a length of the distal collar portion 322. In some embodiments, the distal shaft portion 324 may extend through at least a portion of an interior of the distal collar portion 322.

In some embodiments, as shown in FIG. 7, the central connecting portion 314 is an elongate, hollow shaft that extends between and connects the distal collar portion 322 and the proximal collar portion 316.

In some embodiments, as shown in FIG. 7, the balloon shoulder assembly 302 may further include transition portions which extend between and connect each collar portion to the central connecting portion 314. In some embodiments, the transition portions are formed as one piece with a remainder of the balloon shoulder assembly 302. In some embodiments, the transition portions may be part of the central connecting portion 314 or part of a corresponding collar portion. For example, as shown in FIG. 7, the balloon shoulder assembly 302 includes a proximal transition portion 326 extending between and connecting the proximal collar portion 316 and the central connecting portion 314 and a distal transition portion 328 extending between and connecting the distal collar portion 322 and the central connecting portion 314. The transition portions 326 and 328 may each narrow in diameter from a corresponding collar portion to the central connecting portion 314. In alternate embodiments, the balloon shoulder assembly 302 may not include transition portions or the transition portions may have a different shape than depicted in FIG. 7 (e.g., stepped).

As shown in FIG. 7, each of the proximal collar portion 316 and the distal collar portion 322 have a bulbous shape with a central portion having a largest outer diameter 340 (compared to a remainder of the collar portion) and ends having a smaller diameter that narrows to a diameter of the corresponding shaft portion or central connecting portion 314. In some embodiments, the proximal collar portion 316 and the distal collar portion 322 may be shaped as an ellipsoid. In some embodiments, the proximal collar portion 316 and the distal collar portion 322 may be shaped as a spheroid. The main balloon 108 may be structurally supported by each of the proximal collar portion 316 and the distal collar portion 322, in a region of the central portion with outer diameter 340. In this way, a widest portion of each of the proximal collar portion 316 and the distal collar portion 322, in the radial direction, is at the central portion and the narrowest portion of each of the proximal collar portion 316 and the distal collar portion 322, in the radial direction, is at the ends of the proximal collar portion 316 and the distal collar portion 322.

As introduced above, the balloon shoulder assembly 302 is formed via blow molding. As such, the balloon shoulder assembly 302, including the proximal balloon shoulder 308 and the distal balloon shoulder 312, is hollow and comprises a compressible, blow molded material. The blow molded material may be a polymer, in some embodiments. As explained above and further below, by having balloon shoulders that are hollow and compressible, the balloon shoulders may provide structural support to the main balloon 108 while making them easier to insert within the main balloon 108 and maneuver through a patient's vasculature.

In some embodiments, as shown in FIG. 7, the proximal balloon shoulder 308, the distal balloon shoulder 312, the central connecting portion 314, and the transition portions 326 and 328 (if included) may be molded together and formed as one piece via blow molding. In alternate embodiments, the proximal balloon shoulder 308 and the distal balloon shoulder 312 may be blow molded separately, as two different parts (and not formed as one piece). In some of these embodiments, the balloon shoulder assembly may not include the central connecting portion, and instead, the separately blow molded proximal and distal balloon shoulders may be spaced apart from one another. Thus, in some embodiments, the balloon shoulder assembly 302 may include the proximal balloon shoulder 308 and distal balloon shoulder 312, as shown in FIG. 7, but without the central connecting portion 314. In other of these embodiments, the separately molded proximal balloon shoulder 308 and distal balloon shoulder 312 may be coupled to the central connecting portion 314, after blow molding and before insertion into a main balloon of the balloon catheter.

In some embodiments, the balloon shoulder assembly 302 may be fully enclosed and inflatable (e.g., capable of being inflated from a deflated state). For example, the ends of each of the proximal shaft portion 318 and the distal shaft portion 324 not coupled to the corresponding collar portions, as well as both ends of the collar portions, may be closed. In this way, the balloon shoulder assembly 302 may be completely enclosed and able to contain pressure, thereby allowing it to be inflated. As such, the balloon shoulder assembly 302, or portions of the balloon shoulder assembly (such as the proximal balloon shoulder 308 and distal balloon shoulder 312), may be a balloon (or balloons), in addition to the main balloon 108. Further, in some embodiments where the proximal balloon shoulder and distal balloon shoulder are molded separately, as separate pieces, they each may be additional balloons, in addition to the main balloon 108. In alternate embodiments, the balloon shoulder assembly 302 may not be fully enclosed, and thus may not be able to contain pressure and be inflated (e.g., non-inflatable). Embodiments of blow molded balloon shoulders that are inflatable and embodiments of blow molded balloon shoulders that are non-inflatable are shown in FIGS. 11-13, as described further below.

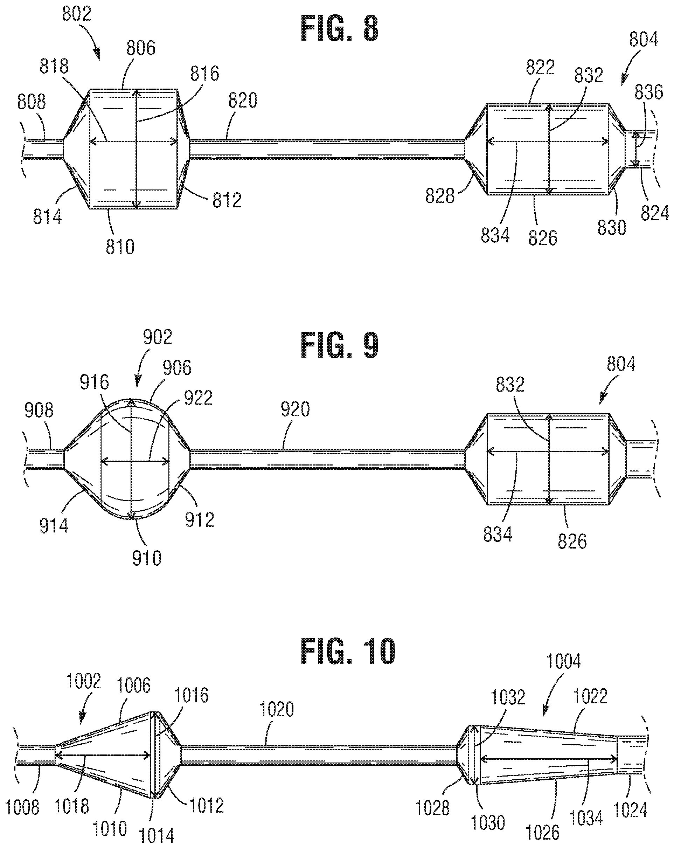

FIGS. 8-10 show alternate embodiments of balloon shoulder assemblies for a balloon catheter, such as balloon catheter 200. Any one of or a combination of the embodiments of FIGS. 8-10 can be used in place of the balloon shoulder assembly 302 in the balloon catheter 200. Each of the embodiments shown in FIGS. 8-10 may be formed as a one-piece balloon shoulder assembly having a central connecting portion connecting proximal and distal portions, or as a balloon shoulder assembly including two, separately molded balloon shoulders (similar to the arrangement of the balloon shoulder assembly along the inner shaft, as shown in FIG. 5, but with blow molded balloon shoulders).

In each of FIGS. 8-10, two, differently shaped and/or sized balloon shoulders are shown connected by a central connecting portion and each including a main body, or collar portion, and a shaft portion. The shaft portion can be affixed to a shaft, nosecone or other component of a delivery apparatus, such as by welding or an adhesive. The collar portion extends radially outward from the shaft portion and is adapted to support the main balloon of the balloon catheter in which the balloon shoulder is inserted. However, the two, differently shaped and/or sized balloon shoulders shown in each of FIGS. 8-10 need not be included in a same balloon shoulder assembly, in some embodiments. Instead, each individual balloon shoulder shown in FIGS. 8-10 may be used as a separately molded balloon shoulder within a balloon shoulder assembly or molded as one-piece with another, similar sized and/or shaped (e.g., a mirror image of the shoulder design shown balloon shoulder and a central connecting portion, as shown in FIGS. 6 and 7. Also, any of individual balloon shoulders shown in FIGS. 8-10 can be used in combination with any of the other balloon shoulders to form a balloon shoulder assembly. For example, a balloon shoulder assembly can comprise a balloon shoulder 802 of FIG. 8, a balloon shoulder 1004 of FIG. 10, and a connecting portion extending therebetween. Moreover, any of the individual balloon shoulders can be used as a proximal balloon shoulder or a distal balloon shoulder in a balloon shoulder assembly.

Turning first to FIG. 8, a first balloon shoulder 802 and a second balloon shoulder 804 are shown. A balloon shoulder assembly can comprise the first balloon shoulder 802, the second balloon shoulder 804, and a central connecting portion 820. In some embodiments, the first balloon shoulder 802 is a distal balloon shoulder and the second balloon shoulder 804 is a proximal balloon shoulder. In other embodiments, the first balloon shoulder 802 is a proximal balloon shoulder and the second balloon shoulder 804 is a distal balloon shoulder.

The first balloon shoulder 802 has a collar portion (e.g., main body) 806 and shaft portion 808. The collar portion 806 includes a central, cylindrical body 810 arranged between a first tapered end portion 812 and a second tapered end portion 814 of the collar portion 806. The cylindrical body 810 has an outer diameter 816, arranged in the radial direction, and a length 818, arranged in the axial direction. As shown in FIG. 8, the outer diameter 816 is larger than the length 818. However, in alternate embodiments, the outer diameter 816 and the length 818 may be the same size or the length 818 may be larger than the outer diameter 816 (e.g., as shown in the embodiment of the second balloon shoulder 804, as described further below).

As shown in FIG. 8, the length 818 of the cylindrical body 810 may be longer than a length of each of the first tapered end portion 812 and the second tapered end portion 814. Each of the first tapered end portion 812 and the second tapered end portion 814 has a wider end directly connected to the cylindrical body 810 and a narrower (e.g., tapered) end. The narrower end of the first tapered end portion 812 is shown connected to a first end of a central connecting portion 820. However, as explained above, in a balloon shoulder assembly for a balloon catheter, a second end of the central connecting portion 820 may be connected to a similarly sized and shaped balloon shoulder to that of first balloon shoulder 802 (instead of the second balloon shoulder 804, as shown in FIG. 8). Further, in some embodiments, the narrower end of the first tapered end portion 812 may not be connected to any central connecting portion, and instead may not be attached to any additional part (e.g., may be open) or may be closed around the inner shaft that the first balloon shoulder 802 is mounted on. The narrower end of the second tapered end portion 814 is directly connected to/around the shaft portion 808.

In some embodiments, lengths (in the axial direction) of the first tapered end portion 812 and the second tapered end portion 814 may be the same. In other embodiments, the length (in the axial direction) of the first tapered end portion 812 may be different than the length of the second tapered end portion 814. For example, as shown in FIG. 8, the length of the second tapered end portion 814 is longer than the length of the first tapered end portion 812.

The second balloon shoulder 804 shown in FIG. 8 has a collar portion (e.g., main body) 822 and a shaft portion 824. The collar portion 822 includes a central, cylindrical body 826 arranged between a first tapered end portion 828 and a second tapered end portion 830 of the collar portion 822. The cylindrical body 826 has an outer diameter 832, arranged in the radial direction, and a length 834, arranged in the axial direction. As shown in FIG. 8, the outer diameter 832 is smaller than the length 834. Thus, compared to the first balloon shoulder 802, the second balloon shoulder 804 has a longer cylindrical body, longer overall length, and a smaller outer diameter.

The shaft portion 824 of the second balloon shoulder 804 also has a larger diameter 836 than the shaft portion 808 of the first balloon shoulder 802. Further, the diameter 836 of the shaft portion 824 is larger than a diameter of the central connecting portion 820. In this way, the diameter of the shaft portion of a balloon shoulder may be larger (or smaller, in some embodiments) than a diameter of the central connecting portion.

As shown in FIG. 8, the length 834 of the cylindrical body 826 may be longer than a length of each of the first tapered end portion 828 and the second tapered end portion 830. Each of the first tapered end portion 828 and the second tapered end portion 830 has a wider end directly connected to the cylindrical body 826 and a narrower (e.g., tapered) end. The narrower end of the first tapered end portion 828 is shown connected to a second end of the central connecting portion 820. However, as explained above, in a balloon shoulder assembly for a balloon catheter, a first end of the central connecting portion 820 may be connected to a similarly sized and shaped balloon shoulder to that of second balloon shoulder 804 (instead of the first balloon shoulder 802, as shown in FIG. 8). Further, in some embodiments, the narrower end of the first tapered end portion 828 may not be connected to any central connecting portion, and instead may not be attached to any additional part (e.g., may be open) or may be closed around the inner shaft that the second balloon shoulder 804 is mounted on. The narrower end of the second tapered end portion 830 is directly connected to the shaft portion 824.

Similar to as explained above for the first balloon shoulder 802, lengths (in the axial direction) of the first tapered end portion 828 and the second tapered end portion 830 of the second balloon shoulder 804 may be the same (in some embodiments) or different (in other embodiments) than one another.

FIG. 9 shows additional balloon shoulder embodiments, including a third balloon shoulder 902. FIG. 9 also shows the second balloon shoulder 804, for comparison with the size and shape of the third balloon shoulder 902. A balloon shoulder assembly can comprise the third balloon shoulder 902, the second balloon shoulder 804, and a central connecting portion 920. In some embodiments, the third balloon shoulder 902 is a distal balloon shoulder and the second balloon shoulder 804 is a proximal balloon shoulder. In other embodiments, the third balloon shoulder 902 is a proximal balloon shoulder and the second balloon shoulder 804 is a distal balloon shoulder.

The third balloon shoulder 902 has a collar portion 906 and a shaft portion 908. The collar portion 906 is bulbous in shape with a curved, central portion 910 positioned between a first tapered end portion 912 and a second tapered end portion 914. A widest portion of the central portion 910 has an outer diameter 916. The central portion 910 then curves inward, relative to a central axis of the third balloon shoulder 902, toward each of the first tapered end portion 912 and the second tapered end portion 914. Each of the first tapered end portion 912 and the second tapered end portion 914 have wider ends coupled to the central portion 910 and narrower ends coupled to a central connecting portion 920 (or in some embodiments, not coupled to a central connecting portion and instead being a closed end or coupled to an inner shaft of the balloon catheter) or the shaft portion 908, respectively.

In some embodiments, as shown in FIG. 9, the first tapered end portion 912 and the second tapered end portion 914 may have different lengths, in the axial direction. In alternate embodiments, the first tapered end portion 912 and the second tapered end portion 914 may have the same length.

As shown in FIG. 9, a length 922 of the central portion 910 is shorter than the length 834 of the cylindrical body 826. However, the outer diameter 916 of the central portion 910, which is a largest diameter of the third balloon shoulder 902, is larger than the outer diameter 832 of the cylindrical body 826, which is a largest diameter of the second balloon shoulder 804.

FIG. 10 shows additional balloon shoulder embodiments, including a fifth balloon shoulder 1002 and a sixth balloon shoulder 1004. The fifth balloon shoulder 1002 and the sixth balloon shoulder 1004 have similar shapes but different sizes and dimensions, as described further below. A balloon shoulder assembly can comprise the fifth balloon shoulder 1002, the sixth balloon shoulder 1004, and a connecting portion 1020. In some embodiments, the fifth balloon shoulder 1002 is a distal balloon shoulder and the sixth balloon shoulder 1004 is a proximal balloon shoulder. In other embodiments, the fifth balloon shoulder 1002 is a proximal balloon shoulder and the sixth balloon shoulder 1004 is a distal balloon shoulder.

Specifically, as shown in FIG. 10, the fifth balloon shoulder 1002 has a collar portion 1006 and a shaft portion 1008. The collar portion 1006 includes an elongate tapered portion 1010, a shorter tapered portion 1012, and a central ring portion 1014 positioned between (and separating) the elongate tapered portion 1010 and the shorter tapered portion 1012. An outer diameter of the collar portion 1006, and the fifth balloon shoulder 1002, is greatest at the central ring portion 1014, as shown by outer diameter 1016. The elongate tapered portion 1010 has a wider end connected to the central ring portion 1014 and then tapers inward, from the wider end to a narrower end connected to the shaft portion 1008. The shorter tapered portion 1012 has a wider end connected to the central ring portion 1014 and then tapers inward, from the wider end to a narrower end connected to a central connecting portion 1020 (or in some embodiments, not coupled to a central connecting portion and instead being a closed end or coupled to an inner shaft of the balloon catheter). The elongate tapered portion 1010 has a length 1018 which may be a longest length of all portions of the collar portion 1006.

The sixth balloon shoulder 1004 has a collar portion 1022 and shaft portion 1024 (which has a larger diameter than shaft portion 1008 of the fifth balloon shoulder 1002). The collar portion 1022 includes an elongate tapered portion 1026, a shorter tapered portion 1028, and a central ring portion 1030 positioned between (and separating) the elongate tapered portion 1026 and the shorter tapered portion 1028. An outer diameter of the collar portion 1022, and the sixth balloon shoulder 1004, is greatest at the central ring portion 1030, as shown by outer diameter 1032. The elongate tapered portion 1026 has a wider end connected to the central ring portion 1030 and then tapers inward, from the wider end to a narrower end connected to the shaft portion 1024. The shorter tapered portion 1028 has a wider end connected to the central ring portion 1030 and then tapers inward, from the wider end to a narrower end connected to a central connecting portion 1020 (or in some embodiments, not coupled to a central connecting portion and instead being a closed end or coupled to an inner shaft of the balloon catheter). The elongate tapered portion 1026 has a length 1034 which may be a longest length of any portion of the collar portion 1022.

As shown in FIG. 10, the length 1034 of the elongate tapered portion 1026, as well as the overall length of the collar portion 1022, of the sixth balloon shoulder are longer than the corresponding components of the fifth balloon shoulder 1002. Further, the outer diameter 1032 of the central ring portion 1030 of the sixth balloon shoulder is smaller than the outer diameter 1016 of the central ring portion 1014 of the fifth balloon shoulder 1002.

The various lengths and/or diameters of the different portions of the fifth and sixth balloon shoulders may be configured for different applications (e.g., different balloon catheter designs and/or sizes). For example, in different embodiments, the length of the elongate tapered portion and/or the lengths and/or outer dimeters of the shorter tapered portion and/or central ring portion of the collar portion of the balloon shoulder may be adjusted based on a size of the main balloon of the balloon catheter, or the size of the balloon catheter itself, in which the balloon shoulder (or balloon shoulder assembly) is adapted to be installed within. Similarly, the dimensions of the various portions of the other balloon shoulder embodiments shown in FIGS. 8-10 may also be adapted based on the intended application or balloon catheter. As introduced above, blow molding the balloon shoulders and/or balloon shoulder assemblies allows for a wider variety of shapes and dimensions to be produced relatively easily and cheaply (as compared to injection molding similar parts). Further, by forming the balloon shoulders via blow molding, hollow balloon shoulders are created (e.g., having outer walls forming hollow shells with void space on an interior, within the outer walls), making the blow molded balloon shoulders less bulky and having reduced mass compared to injection molded shoulders. As a result of this reduced mass, the overall length of the blow molded balloon shoulders may be reduced, thereby allowing the delivery system (e.g., the balloon catheter) to be shorter.

In some embodiments, as shown in FIGS. 8-10, the blow molded balloon shoulders and balloon shoulder assemblies may be closed (sealed), and thus, inflatable. For example, the balloon shoulders (if formed separately) and/or the balloon shoulder assemblies (if formed as one piece) may be fully enclosed, with all ends and walls closed/sealed, such that pressure (air or liquid) may be contained within an interior of the balloon shoulders and/or balloon shoulder assemblies. For example, if the balloon shoulder assembly is formed as one piece with a central connecting portion, ends of the shaft portions of each of the proximal and distal balloon shoulders may be fully closed (sealed), such as depicted in the embodiments of FIGS. 6-7. In this way, inflating the balloon shoulders and/or balloon shoulder assemblies with fluid (air or liquid) may cause walls of the balloon shoulders and/or balloon shoulder assemblies to expand (e.g., as a balloon). Thus, these inflatable balloon shoulders and/or balloon shoulder assemblies may have a deflated (not inflated) state and an inflated state.

FIGS. 11-13 show embodiments of inflatable and non-inflatable, blow molded balloon shoulder assemblies. Specifically, FIG. 11 shows an embodiment of an inflatable balloon shoulder assembly 1100 in a non-inflated (e.g., deflated) state, FIG. 12 shows the embodiment of the inflatable balloon shoulder assembly 1100 in an inflated state, and FIG. 13 shows an embodiment of a non-inflatable balloon shoulder assembly 1300. The balloon shoulder assemblies shown in FIGS. 11-13 may be inserted within a main balloon of a balloon catheter, such as the balloon catheter 200 shown in FIGS. 6 and 7. Additionally, as shown in FIGS. 11-13, the balloon shoulder assemblies are formed as one piece, including a central connecting portion. However, in alternate embodiments, the proximal and distal balloon shoulders of the balloon shoulder assemblies shown in FIGS. 11-13 may be formed separately, without the central connecting portion, and separately installed within a main balloon of a balloon catheter. In yet other embodiments, the proximal and distal balloon shoulders and the central connecting portion of FIGS. 11-13 may all be formed separately (via blow molding) and then coupled together, after being blow molded, as a balloon shoulder assembly.

Turning first to FIGS. 11 and 12, the inflatable balloon shoulder assembly 1100 includes a proximal balloon shoulder 1102, a distal balloon shoulder 1104, and a central connecting portion 1106. The proximal balloon shoulder 1102 includes a collar portion 1108 and a shaft portion 1110, where a proximal end 1112 of the shaft portion 1110 forms a proximal end of the proximal balloon shoulder 1102 and a distal end 1114 of the collar portion 1108 forms a distal end of the proximal balloon shoulder 1102. Each of the distal end 1114 and the proximal end 1112 are closed ends (e.g., having enclosed walls for pressure containment). The shaft portion 1110 may extend through a center of the collar portion 1108 and connect to or be continuous with (when formed as one piece) the central connecting portion 1106. As shown in FIG. 11, the collar portion 1108 is conical in shape with a wider end arranged at the distal end 1114 and a narrower end arranged closer to the proximal end 1112. However, in alternate embodiments, the collar portion 1108 may have a different shape, such as one of the shapes described above with reference to FIGS. 6-10.

The distal balloon shoulder 1104 has a similar (e.g., same) shape and arrangement as the proximal balloon shoulder 1102. Specifically, the distal balloon shoulder includes a collar portion 1116 and a shaft portion 1118 that extends outward from and through the collar portion 1116, along a central axis of the inflatable balloon shoulder assembly 1100. A proximal end 1120 of the collar portion 1116 forms a proximal end of the distal balloon shoulder 1104 and a distal end 1122 of the shaft portion 1118 forms a distal end of the distal balloon shoulder 1104. Each of the distal end 1122 and the proximal end 1120 are closed ends (e.g., having walls for pressure containment).

Thus, due to the closed ends and fully sealed portions of the balloon shoulders, as described above, the inflatable balloon shoulder assembly 1100 is configured to be inflated. Specifically, one or more portions of the inflatable balloon shoulder assembly 1100 may receive and retain an inflating fluid (e.g., air or liquid) in order to inflate (e.g., expand outward relative to the central axis of the inflatable balloon shoulder assembly 1100).

For example, as shown in FIG. 12, the enclosed proximal balloon shoulder 1102 and the enclosed distal balloon shoulder 1104 are inflated. As such, the inflatable balloon shoulder assembly 1100 is in its inflated state. As shown in FIG. 12, outer walls 1124 of each of the proximal balloon shoulder 1102 and the distal balloon shoulder 1104 expand outward as an interior of each of the proximal balloon shoulder 1102 and the distal balloon shoulder 1104 receive an inflating fluid and become inflated. As a result, a largest (e.g., maximal) outer diameter 1126 of each of the proximal balloon shoulder 1102 and the distal balloon shoulder 1104 increases relative to the non-inflated state shown in FIG. 11.

In the inflated state, as shown in FIG. 12, the balloon shoulders of the balloon shoulder assembly may provide structural support to a main balloon of a balloon catheter and form ends (e.g., shoulders) of a valve retaining portion of the main balloon. As a result, a prosthetic medical device, such as a prosthetic valve, crimped around the valve retaining portion of the main balloon may be held in place and not move, axially, past either of the proximal and distal balloon shoulders. In some embodiments, after deployment of the prosthetic medical device from the balloon catheter, the balloon shoulders may be deflated, into the deflated state shown in FIG. 11, for example, to aid in removal of the balloon catheter from a patient's body.

In some embodiments, as shown in FIG. 13, the balloon shoulder assembly may be configured to provide structural support to the main balloon of the balloon catheter without being inflatable. For example, FIG. 13 shows a blow molded, non-inflatable balloon shoulder assembly 1300 including a proximal balloon shoulder 1302 and a distal balloon shoulder 1304 which are each blow molded and not inflatable (e.g., not configured to be inflated, as explained further below). The balloon shoulder assembly 1300 additionally includes a central connecting portion 1306 arranged between and connecting the proximal balloon shoulder 1302 and the distal balloon shoulder 1304. However, in alternate embodiments, the balloon shoulder assembly 1300 may not include the central connecting portion 1306 and the proximal balloon shoulder 1302 and the distal balloon shoulder 1304 may be formed separately as two pieces. Each of the proximal balloon shoulder 1302 and the distal balloon shoulder 1304 includes a collar portion 1308 and a shaft portion 1310. The shaft portion 1310 may run through a center of the collar portion 1308 and be continuous with or connect to the central connecting portion 1306, at an innermost end of the collar portion 1308. Alternately, in some embodiments, the shaft portion 1310 may connect to an outermost, narrower end of the collar portion 1308, and the central connecting portion 1306 may extend to the narrower end of the collar portion 1308.

As shown in FIG. 13, the collar portion 1308 has a funnel shape with tapered outer walls 1312 which extend between an open, wider end 1314 and a narrower end 1316 of the collar portion 1308. In alternate embodiments, the collar portion 1308 may have a different shape, such as one of the shapes shown in the embodiments of FIGS. 6-10, as described above. The narrower end 1316 may close around the shaft portion 1310, but at least the wider end 1314 is open and not enclosed around the shaft portion 1310 or the central connecting portion 1306. As such, the proximal balloon shoulder 1302 and the distal balloon shoulder 1304 are not fully enclosed and capable of retaining fluid pressure, and thus, are not inflatable. The outer walls 1312 may have a thickness that provides structural support to a main balloon surrounding the balloon shoulder assembly 1300 (when inserted within the main balloon of a balloon catheter). For example, the outer walls 1312 may have an increased thickness relative to outer walls of an inflatable balloon shoulder assembly (such as the inflatable balloon shoulder assembly 1100 shown in FIGS. 11 and 12). In some embodiments, the increased thickness of the outer walls 1312 can be in a range of .001 inch to .01 inch. In some embodiments, the wall thickness of an inflatable shoulder (including any of the embodiments described above) can be in the range of .0001 inch to .01 inch. The increased wall thickness of the outer walls 1312 allows of the balloon shoulders of the balloon shoulder assembly 1300 to provide structural support to the main balloon of the balloon catheter without inflation.

In this way, a blow molded balloon shoulder assembly for a balloon catheter may be configured to be inflatable or non-inflatable, as shown in FIGS. 11-13. Further, the balloon shoulder assembly may be formed as one piece, including both the proximal and distal balloon shoulders, or as two or more pieces (e.g., the proximal and distal balloon shoulders may be formed separately and not integrally formed with one another). Further still, as shown in FIGS. 6-10, the balloon shoulders of the balloon shoulder assembly may have different shapes and/or sizes. The different shapes and/or sizes of the balloon shoulders may be selected and adapted based on a type, size, and/or shape of the delivery system (e.g., main balloon and/or balloon catheter) into which they are intended to be inserted and used. Moreover, in other embodiments, a balloon shoulder assembly can include an inflatable shoulder (including any of the embodiments described above) and a non-inflatable shoulder (including any of the embodiments described above), which optionally can be connected by a connecting portion.

In still other embodiments, a balloon shoulder assembly can include a single shoulder, such as a single inflatable shoulder (including any of the embodiments described above) or a single non-inflatable shoulder (including any of the embodiments described above). The single shoulder can be used as a proximal shoulder or a distal shoulder and can include a connecting portion (e.g., central connecting portion 214), although the connecting portion in such embodiments serves as a mounting portion for the single shoulder, since it does not connect two shoulders. The mounting portion can be affixed to a shaft or other component of the delivery apparatus, such as by welding or an adhesive. In other implementations, the mounting portion need not be used and the single shoulder can be mounted directly on a component of the balloon catheter, such as the inner shaft 106, the outer shaft 104, or the nosecone 110.

In some embodiments, the prosthetic valve can be initially crimped on the delivery apparatus at a location offset from the main balloon 108 and then slid onto the balloon after being inserted into the patient's vasculature, such as disclosed in U.S. Publication Nos. 2009/0281619 and 2013/0030519. For example, the prosthetic valve can be initially crimped onto the delivery apparatus at a location proximal to the balloon 108 (such as on the outer shaft 104), and then slide onto the balloon 108 after being inserted into the patient's vasculature. In such cases, the shoulder assembly can include a single shoulder, or one of the shoulders can be much smaller than the other, to facilitate positioning of the crimped prosthetic valve onto the balloon 108 inside the patient's body. For example, in one specific implementation, the shoulder assembly can include single shoulder, which can be a distal shoulder and the prosthetic valve can be initially crimped at a location proximal to the main balloon 108. In another implementation, the single shoulder can be a proximal shoulder and the prosthetic valve can be initially crimped at a location distal to the main balloon 108.

FIG. 14 shows a flow chart of a method 1400 for manufacturing (e.g., forming) a balloon catheter including a blow molded balloon shoulder assembly. The balloon shoulder assembly may be one of the balloon shoulder assemblies described herein with reference to FIGS. 6-13. The balloon catheter may be similar to one or more of the balloon catheters described herein with reference to FIGS. 3-7. In some embodiments, the balloon catheter may be part of a delivery system (e.g., endovascular delivery system) configured to deliver a prosthetic medical device to a target location within a body of a patient, such as a transcatheter heart valve delivery system. As described above, the blow molded balloon shoulders of the balloon shoulder assembly may be configured to support a main balloon of a balloon catheter in which they are installed and form a device sitting pocket on the main balloon to reduce movement (e.g., axial movement) of the prosthetic medical device during an insertion (e.g., implantation) process.

Method 1400 begins at 1402 by forming a balloon shoulder assembly via blow molding. The balloon shoulder assembly may include a proximal balloon shoulder and a distal balloon shoulder spaced apart from one another, in an axial direction relative to a central axis of the balloon shoulder assembly. In some embodiments, the proximal balloon shoulder and the distal balloon shoulder may be blow molded as one piece with a central connecting portion arranged between the proximal and distal balloon shoulders. In other embodiments, the proximal balloon shoulder and the distal shoulder may be blow molded as separate pieces.

Further, in some embodiments, blow molding the balloon shoulder assembly may include forming a fully inflatable balloon shoulder assembly that is adapted to contain pressure (e.g., fluid pressure). In these embodiments, at least the proximal balloon shoulder and the distal balloon shoulder of the balloon shoulder assembly (and in some embodiments, the entire balloon shoulder assembly) may be fully enclosed with ends that are adapted to be sealed around one or more shafts of the balloon catheter during assembly. An example of a fully enclosed, inflatable balloon shoulder assembly formed via blow molding is shown in FIGS. 11-12, as described above.

In other embodiments, blow molding the balloon shoulder assembly may include forming an at least partially open and not inflatable balloon shoulder assembly. For example, at least one end or portion of the proximal balloon shoulder and/or the distal balloon shoulder may be open such that fluid pressure may escape or not be fully contained, even after installing on one or more shafts of the balloon catheter. An example of a non-inflatable balloon shoulder assembly formed via blow molding is shown in FIG. 13, as described above.

As introduced above, blow molding the balloon shoulder assembly at 1402 results in a balloon shoulder assembly with compressible, outer walls and a hollow interior. Blow molding the balloon shoulder assembly 1402 may include blow molding a balloon shoulder assembly with outer walls of a desired (e.g., pre-set) thickness. The thickness may be selected to be thicker for non-inflatable balloon shoulder assemblies and thinner for inflatable balloon shoulder assemblies. Further, in some embodiments, blow molding the balloon shoulder assembly at 1402 may include blow molding the balloon shoulder assembly to have balloon shoulders of a pre-determined size and/or shape. As described above, blow molding the balloon shoulder assembly may allow for balloon shoulder assemblies with a wider variety of shapes and/or sizes to be created more cheaply and easily (as compared to injection molding).

Method 1400 continues at 1404 and includes mounting the blow molded balloon shoulder assembly to one or more shafts of the delivery system (e.g., the balloon catheter). In some embodiments, the method at 1404 may include mounting an entirety of the blow molded balloon shoulder to one or more shafts or other components of the balloon catheter (e.g., the inner shaft 106 shown in FIG. 5). In one specific implementation, the entire balloon shoulder assembly, including the proximal balloon shoulder and the distal balloon shoulder, may be mounted on the innermost shaft of a delivery apparatus (e.g., inner shaft 106 shown in FIGS. 4 and 6-7).

In another implementation, as depicted in FIG. 15A, a balloon shoulder assembly 1500 can be arranged such that a proximal shoulder 1502 is mounted to an outer shaft 104 of a delivery apparatus and a distal shoulder 1504 is mounted to an inner shaft 106 of the delivery apparatus. In another implementation, as depicted in FIG. 15B, a balloon shoulder assembly 1600 can be arranged such that a proximal shoulder 1602 is mounted to an outer shaft 104 of a delivery apparatus and a distal shoulder 1604 is mounted to a nosecone 110 of the delivery apparatus. In another implementation, as depicted in FIG. 15C, a balloon shoulder assembly 1700 can be arranged such that a proximal shoulder 1702 is mounted to an inner shaft 106 of a delivery apparatus and a distal shoulder 1604 is mounted to a nosecone 110 of the delivery apparatus. It should be understood that the shoulder assemblies 1500, 1600, and 1700 can represent any of the shoulder assembly embodiments disclosed herein; that is, any of the shoulder assembly embodiments disclosed herein can be mounted to a delivery apparatus in any of the ways shown in FIGS. 15A-15C.

At 1406, method 1400 includes positioning the blow molded balloon shoulder assembly inside the main balloon of the delivery system (e.g., balloon catheter). In some embodiments, the main balloon may be similar to the main balloon 108 shown in FIGS. 3-7. For example, in some embodiments, the method at 1406 may include inserting the blow molded balloon shoulder assembly, mounted on the one or more shafts of the balloon catheter, into the main balloon such that the main balloon surrounds at least a portion of the balloon shoulder assembly (e.g., at least the proximal and distal balloon shoulders). In some embodiments, if the balloon shoulder assembly is inflatable (e.g., adapted to be inflated via fluid pressure), the method at 1406 includes inserting the blow molded balloon shoulder assembly, in its deflated state, into the main balloon. As such, it may be easier to insert the non-inflated proximal and distal balloon shoulders into the main balloon (e.g., with reduced resistance as compared to injection molded balloon shoulders). Further, in some embodiments, even if not inflated, the blow molded balloon shoulder assembly may be easier to insert into the main balloon, compared to injection molded balloon shoulder assemblies, due to the increased compressibility and hollow nature of the blow molded balloon shoulder assembly. For example, the outer walls of the blow molded balloon shoulder assembly may compress as they are pushed and inserted into the main balloon and then expand back into a non-compressed state after insertion into the main balloon. Such flexible (temporary) deformation may not be possible with injection molded balloon shoulder assemblies.

Method 1400 may optionally proceed to 1408 to inflate the balloon shoulder assembly, or a portion of the balloon shoulder assembly. For example, after positioning the balloon shoulder assembly into the main balloon, if the balloon shoulder assembly is configured to be inflated (e.g., being enclosed to contain pressure and thus inflatable), the method at 1408 may optionally include inflating the balloon shoulder assembly from a non-inflated (e.g., deflated) to an inflated state. In some embodiments, inflating the balloon shoulder assembly may include inflating the proximal balloon shoulder and the distal balloon shoulder assembly such that their outer walls expand outward and push against walls of the main balloon. In some embodiments, inflating the balloon shoulder assembly may include delivering a fluid to an interior of the balloon shoulder assembly (e.g., an interior of the proximal and distal balloon shoulders). In some embodiments, the fluid may be saline, a contrast mixture, or another type of biocompatible media. In other embodiments, the fluid may be a curable or non-curable material that may make the balloon shoulders solid pieces. After inflating the balloon shoulder assembly, the main balloon (e.g., balloon 108) may be pleated and folded around the contours of the balloon shoulder assembly so that it is ready to receive a prosthetic valve. In alternative embodiments, inflating the balloon shoulder assembly 1408 may not occur until the assembled balloon catheter is delivered to an end user. For example, the balloon shoulder assembly, within the balloon catheter, may be delivered to the end user in the deflated state and then inflated by the user just prior to positioning the prosthetic medical device (e.g., prosthetic valve) on the main balloon of the balloon catheter.

However, in some embodiments, when the blow molded balloon shoulder assembly is not configured to be inflated, method 1400 may proceed directly from 1406 to 1410. At 1410, the method includes crimping a prosthetic medical device (e.g., a prosthetic heart valve) on a device retaining portion of the main balloon of the balloon catheter, formed between the proximal balloon shoulder and the distal balloon shoulder of the blow molded balloon shoulder assembly. In one embodiment, the prosthetic medical device may be crimped onto the balloon catheter during assembly, and prior to shipping the assembled balloon catheter to the end user. In another embodiment, the assembled balloon catheter, without a prosthetic medical device crimped thereon, may be shipped to the end user. After receiving the assembled balloon catheter and prior to a procedure, the end user may then crimp the prosthetic medical device onto the device retaining portion of the main balloon of the balloon catheter.

As explained above, inner ends (e.g., the ends facing each other) of collar portions of the proximal balloon shoulder and the distal balloon shoulder may structurally support the main balloon and form a natural pocket, in the space separating the inner ends, for the prosthetic medical device to be crimped. As a result, during an implantation procedure where the balloon catheter is pushed through a lumen of a patient, the prosthetic medical device may remain within the device retaining portion and not move axially past the proximal or distal balloon shoulders. As explained above, in some embodiments, after the prosthetic medical device is deployed (after inflating the main balloon) and implanted in the patient), the balloon shoulders of the balloon shoulder assembly may be deflated (if inflated at 1408 and/or during insertion) to decrease the resistance of retrieving the balloon catheter from inside the patient.

In this way, a balloon shoulder assembly for a balloon catheter of a delivery system may include a proximal balloon shoulder including a collar portion and a shaft portion and a distal balloon shoulder including a collar portion and a shaft portion. Each of the proximal balloon shoulder and the distal balloon shoulder may be hollow and comprise a compressible, blow molded material. The proximal and distal balloon shoulders may be spaced apart from one another, in an axial direction relative to a central axis of the balloon catheter, within a main balloon of a balloon catheter. As such, a device (e.g., valve) retaining portion of the main balloon is created and adapted to receive a prosthetic medical device. In some embodiments, the delivery system is a transcatheter heart valve delivery system and the prosthetic medical device is a prosthetic heart valve. In some embodiments, the balloon shoulder assembly may include a central connecting portion arranged between the proximal and distal balloon shoulders and the entire balloon shoulder assembly may be formed as one piece. In other embodiments, the balloon shoulder assembly may not be formed as one piece and the proximal and distal balloon shoulders may be individually blow molded as separated pieces.

By forming the balloon shoulder assembly via blow molding, hollow, compressible balloon shoulders are generated that may have reduced hardness, reduced mass, reduced stiffness, increased compressibility, and comparable structural strength as compared to similar, injection molded balloon shoulder assemblies. As a result, blow molded balloon shoulder assemblies may be more resilient, allowing them to compress and pop back into an expanded shape, while still providing ample structural support to the main balloon of the balloon catheter, thereby making them easier to insert within the main balloon of the balloon catheter and reducing degradation to a sheath during maneuvering the balloon catheter through the sheath during an implantation procedure. Further, a balloon catheter including the blow molded balloon shoulders and/or balloon shoulder assembly may be easier to retrieve (e.g., with reduced force or resistance) through the sheath and/or patient's vasculature after implantation of the prosthetic medical device. Further still, blow molding may make it possible to change a design of the balloon shoulder assembly, such as the size and/or shape, more easily and cheaply than injection molding (e.g., due to the expensive nature of the molds required for injection molding).

Additionally, in some embodiments, the balloon shoulder assembly may be inflatable (and exist either in a deflated or inflated state). In other embodiments, the balloon shoulder assembly may be non-inflatable. Inflatable balloon shoulders may further increase the ease of retrieval of the balloon catheter after implantation of the prosthetic medical device. For example, after implantation of the prosthetic medical device and during retrieval of the balloon catheter from the implantation site, the balloon shoulders may be deflated, thereby reducing a resistance of the balloon catheter against a sheath or vasculature of the patient as it is pulled out of the patient.

Further, in still other embodiments, any of the balloon shoulder assemblies disclosed herein can be made using manufacturing techniques other than blow molding, including but not limited to, injection molding, dipping, compression molding, etc.

### General Considerations

It should be understood that the disclosed embodiments can be adapted to deliver and implant prosthetic devices in any of the native annuluses of the heart (e.g., the pulmonary, mitral, and tricuspid annuluses), and can be used with any of various delivery approaches (e.g., retrograde, antegrade, transseptal, transventricular, transatrial, etc.).

For purposes of this description, certain aspects, advantages, and novel features of the embodiments of this disclosure are described herein. The disclosed methods, apparatus, and systems should not be construed as being limiting in any way. Instead, the present disclosure is directed toward all novel and nonobvious features and aspects of the various disclosed embodiments, alone and in various combinations and sub-combinations with one another. The methods, apparatus, and systems are not limited to any specific aspect or feature or combination thereof, nor do the disclosed embodiments require that any one or more specific advantages be present or problems be solved. The technologies from any example can be combined with the technologies described in any one or more of the other examples. In view of the many possible embodiments to which the principles of the disclosed technology may be applied, it should be recognized that the illustrated embodiments are only preferred examples and should not be taken as limiting the scope of the disclosed technology.

Although the operations of some of the disclosed embodiments are described in a particular, sequential order for convenient presentation, it should be understood that this manner of description encompasses rearrangement, unless a particular ordering is required by specific language set forth below. For example, operations described sequentially may in some cases be rearranged or performed concurrently. Moreover, for the sake of simplicity, the attached figures may not show the various ways in which the disclosed methods can be used in conjunction with other methods. Additionally, the description sometimes uses terms like "provide" or "achieve" to describe the disclosed methods. These terms are high-level abstractions of the actual operations that are performed. The actual operations that correspond to these terms may vary depending on the particular implementation and are readily discernible by one of ordinary skill in the art.

As used herein, with reference to the transcatheter delivery system, the balloon catheter, the introducer sheath (e.g., the sheath), the balloon shoulder assembly, and the balloon shoulders, "proximal" refers to a position, direction, or portion of a component that is closer to a handle of the delivery system that is outside the patient, while "distal" refers to a position, direction, or portion of a component that is further away from the handle. The terms "longitudinal" and "axial" refer to an axis extending in the proximal and distal directions, unless otherwise expressly defined.

As used in this application and in the claims, the singular forms "a," "an," and "the" include the plural forms unless the context clearly dictates otherwise. Additionally, the term "includes" means "comprises." Further, the terms "coupled" and "connected" generally mean electrically, electromagnetically, and/or physically (e.g., mechanically or chemically) coupled or linked and does not exclude the presence of intermediate elements between the coupled or associated items absent specific contrary language.

Directions and other relative references (e.g., inner, outer, upper, lower, etc.) may be used to facilitate discussion of the drawings and principles herein, but are not intended to be limiting. For example, certain terms may be used such as "inside," "outside,", "top," "down," "interior," "exterior," and the like. Such terms are used, where applicable, to provide some clarity of description when dealing with relative relationships, particularly with respect to the illustrated embodiments. Such terms are not, however, intended to imply absolute relationships, positions, and/or orientations. For example, with respect to an object, an "upper" part can become a "lower" part simply by turning the object over. Nevertheless, it is still the same part and the object remains the same. As used herein, "and/or" means "and" or "or," as well as "and" and "or."

## Claims

1. A balloon shoulder assembly (118; 202; 302; 1100; 1300; 1500; 1600; 1700) for a balloon catheter (100; 200), comprising:
a proximal balloon shoulder (120; 208; 308; 802; 1002; 1102;, 1302; 1502; 1602; 1702) including a proximal collar portion (216; 316; 806; 1006; 1108) that extends radially outward from a proximal shaft portion, relative to a central axis of the balloon shoulder assembly (118; ... 1700); and
a distal balloon shoulder (122; 212; 312; 804; 902; 1004; 1104; 1304; 1504; 1604; 1704) including a distal collar portion (222; 322; 1022; 1116) that extends radially outward from a distal shaft portion (224; 324), wherein each of the proximal balloon shoulder (120; ... 1702) and the distal balloon shoulder (122; ... 1704) are hollow and comprise a compressible, blow molded material and are configured to be positioned within and support an inflatable, main balloon (108) of the balloon catheter (100; 200).

2. The balloon shoulder assembly (118; ... 1700) of claim 1, wherein outer walls of the proximal balloon shoulder (120; ... 1702) and outer walls of the distal balloon shoulder (122; ... 1704) are configured to compress or stretch under applied pressure and return back to an uncompressed or unstretched state upon removal of the applied pressure.

3. The balloon shoulder assembly (118; ... 1700) of any of the preceding claims, wherein the proximal collar portion (216; ... 1108) is spaced away from the distal collar portion (222; ... 1116), in an axial direction relative to the central axis (220).

4. The balloon shoulder assembly (118; ... 1700) of any of the preceding claims, wherein the proximal balloon shoulder (120; ... 1702) and the distal balloon shoulder (122; ... 1704) are molded as one piece with a central connecting portion (214; 314; 820; 920; 1020; 1106; 1306), the central connecting portion (214; ... 1306) arranged between and connecting the proximal collar portion (216; ... 1108) and the distal collar portion (222; ... 1116), wherein, preferably, the proximal balloon shoulder (120; ... 1702) further includes a proximal transition portion (226; 326) extending between and connecting the proximal collar portion (216; ... 1108) and the central connecting portion (214; ... 1306), the proximal transition portion narrowing in diameter from the proximal collar portion (216; ... 1108) to the central connecting portion (214; ... 1306) and wherein the distal balloon shoulder (122; ... 1704) further includes a distal transition portion (228; 328) extending between and connecting the distal collar portion (222; ... 1116) and the central connecting portion (214; ... 1306), the distal transition portion narrowing in diameter from the distal collar portion (222; ... 1116) to the central connecting portion (214; ... 1306).

5. The balloon shoulder assembly (118; ... 1700) of any of claims 1 to 3, wherein the proximal balloon shoulder (120; ... 1702) and the distal balloon shoulder (122; ... 1704) are molded separately, as two separate pieces.

6. The balloon shoulder assembly (118; ... 1700) of any of the preceding claims, wherein each of the proximal balloon shoulder (120; ... 1702) and the distal balloon shoulder (122; ... 1704) are inflatable.

7. The balloon shoulder assembly (118; ... 1700) of any of the preceding claims, wherein the proximal balloon shoulder (120; ... 1702) and the distal balloon shoulder (122; ... 1704) are not fluidly coupled with one another and are individually inflatable.

8. The balloon shoulder assembly (118; ... 1700) of any of claims 1 to 5, wherein each of the proximal balloon shoulder (120; ... 1702) and the distal balloon shoulder (122; ... 1704) are not inflatable, wherein, preferably, each of the proximal balloon shoulder (120; ... 1702) and the distal balloon shoulder (122; ... 1704) have a wall thickness in a range of 0.000254 cm to 0.0254 cm and/or, wherein, preferably, the proximal collar portion (216; ... 1108) has an open end facing an open end of the distal collar portion (222; ... 1116).

9. The balloon shoulder assembly (118; ... 1700) of any of the preceding claims, wherein each of the proximal collar portion (216; ... 1108) and the distal collar portion (222; ... 1116) are funnel-shaped with a wider end (230) that flares radially outward from a narrower end (232), wherein the narrower end (232) of the proximal collar portion (216; ... 1108) is connected to the proximal shaft portion (218; 318), wherein the narrower end (232) of the distal collar portion (222; ... 1116) is connected to the distal shaft portion (224; 324), and wherein the wider ends (230) of each of the proximal collar portion (216; ... 1108) and the distal collar portion (222; ... 1116) face one another and are arranged normal to the central axis.

10. The balloon shoulder assembly (118; ... 1700) of any of claims 1 to 8, wherein each of the proximal collar portion (216; ... 1108) and the distal collar portion (222; ... 1116) have a bulbous shape with a wider, central portion (340) that narrows to two opposite ends.

11. The balloon shoulder assembly (118; ... 1700) of any of claims 1 to 8, wherein one or more of the proximal collar portion (216; ... 1108) and the distal collar portion (222; ... 1116) includes a central, cylindrical body (810; 826; 910) arranged between a first tapered end portion (812; 828; 912) and a second tapered end portion (814; 830; 914), the first tapered end portion (812; 828; 912) and the second tapered end portion (814; 830; 914) tapering, in opposite directions, from the central, cylindrical body (810; 826; 910) to one of the central connecting portion (214; ... 1306) or a corresponding one of the proximal shaft portion or the distal shaft portion (224; 324).

12. The balloon shoulder assembly (118; ... 1700) of any of claims 1 to 8, wherein one or more of the proximal collar portion (216; ... 1108) and the distal collar portion (222; ... 1116) includes a curved, central portion positioned between a first tapered end portion (812; 828; 912) and a second tapered end portion (814; 830; 914), the first tapered end portion (812; 828; 912) and the second tapered end portion (814; 830; 914) tapering, in opposite directions, from the central portion (810; 826; 910) to one of the central connecting portion (214; ... 1306) or a corresponding one of the proximal shaft portion or the distal shaft portion (224; 324).

13. The balloon shoulder assembly (118; ... 1700) of any of claims 1 to 8, wherein one or more of the proximal collar portion (1006) and the distal collar portion (1022) includes an elongate tapered portion (1010; 1026), a shorter tapered portion (1012; 1028), and a central ring portion (1014; 1030) positioned between the elongate tapered portion (1010; 1026) and the shorter tapered portion (1012; 1028).

14. A method of manufacturing a balloon catheter (100; 200), comprising:
blow molding a balloon shoulder assembly (118; ... 1700) including a proximal balloon shoulder (120; ... 1702) and a distal balloon shoulder (122; ... 1704); and
installing the blow molded balloon shoulder assembly (118; ... 1700) in the balloon catheter (100; 200) by positioning the balloon shoulder assembly (118; ... 1700) within an inflatable, main balloon (108) of the balloon catheter (100; 200).

15. A balloon catheter (100; 200) for an endovascular delivery system, comprising:
a proximal balloon shoulder (120; ... 1702) mounted on an inner shaft of the balloon catheter (100; 200), the proximal balloon shoulder (120; ... 1702) comprising a compressible, hollow shell;
a distal balloon shoulder (122; ... 1704) mounted on the inner shaft, the distal balloon shoulder (122; ... 1704) comprising a compressible, hollow shell; and
an inflatable, main balloon (108) that encloses the proximal balloon shoulder (120; ... 1702) and the distal balloon shoulder (122; ... 1704).

## Patentansprüche

1. Ballonschulteranordnung (118; 202; 302; 1100; 1300; 1500; 1600; 1700) für einen Ballonkatheter (100; 200), umfassend:
eine proximale Ballonschulter (120; 208; 308; 802; 1002; 1102; 1302; 1502; 1602; 1702), die einen proximalen Kragenabschnitt (216; 316; 806; 1006; 1108) einschließt, der sich von einem proximalen Schaftabschnitt relativ zu einer Zentralachse der Ballonschulteranordnung (118; ... 1700) auswärts erstreckt; und
eine distale Ballonschulter (122; 212; 312; 804; 902; 1004; 1104; 1304; 1504; 1604; 1704), die einen distalen Kragenabschnitt (222; 322; 1022; 1116) einschließt, der sich von einem distalen Schaftabschnitt (224; 324) radial auswärts erstreckt, wobei jede von der proximalen Ballonschulter (120; ... 1702) und der distalen Ballonschulter (122; ... 1704) hohl ist und ein komprimierbares blasgeformtes Material einschließt, und ausgestaltet ist, um innerhalb eines befüllbaren Hauptballons (108) des Ballonkatheters (100; 200) positioniert zu werden und diesen zu stützen.

2. Ballonschulteranordnung (118; ... 1700) nach Anspruch 1, wobei Außenwände der proximalen Ballonschulter (120; ... 1702) und Außenwände der distalen Ballonschulter (122; ... 1704) so ausgestaltet sind, dass sie unter ausgeübtem Druck komprimiert oder gereckt werden und bei Entfernung des ausgeübten Drucks wieder in einen unkomprimierten oder ungereckten Zustand zurückkehren.

3. Ballonschulteranordnung (118; ... 1700) nach einem der vorhergehenden Ansprüche, wobei der proximale Kragenabschnitt (216; ... 1108) von dem distalen Kragenabschnitt (222; ... 1116) in einer axialen Richtung relativ zu der Zentralachse (220) beabstandet ist.

4. Ballonschulteranordnung (118; ... 1700) nach einem der vorhergehenden Ansprüche, wobei die proximale Ballonschulter (120; ... 1702) und die distale Ballonschulter (122; ... 1704) einteilig mit einem zentralen Verbindungsabschnitt (214; 314; 820; 920; 1020; 1106; 1306) geformt sind, wobei der zentrale Verbindungsabschnitt (214; ... 1306) zwischen dem proximalen Kragenabschnitt (216; ... 1108) und dem distalen Kragenabschnitt (222; ... 1116) angeordnet ist und diese verbindet, wobei vorzugsweise die proximale Ballonschulter (120; ... 1702) des Weiteren einen proximalen Übergangsabschnitt (226; 326) einschließt, der sich zwischen dem proximalen Kragenabschnitt (216; ... 1108) und dem zentralen Verbindungsabschnitt (214; ... 1306) erstreckt und diese verbindet, wobei der proximale Übergangsabschnitt im Durchmesser von dem proximalen Kragenabschnitt (216; ... 1108) zu dem zentralen Verbindungsabschnitt (214; ... 1306) schmaler wird, und wobei die distale Ballonschulter (122; ... 1704) des Weiteren einen distalen Übergangsabschnitt (228; 328) einschließt, der sich zwischen dem distalen Kragenabschnitt (222; ... 1116) und dem zentralen Verbindungsabschnitt (214; ... 1306) erstreckt und diese verbindet, wobei der distale Übergangsabschnitt im Durchmesser von dem distalen Kragenabschnitt (222; ... 1116) zu dem zentralen Verbindungsabschnitt (214; ... 1306) schmaler wird.

5. Ballonschulteranordnung (118; ... 1700) nach einem der Ansprüche 1 bis 3, wobei die proximale Ballonschulter (120; ... 1702) und die distale Ballonschulter (122; ... 1704) separat als zwei separate Teile geformt werden.

6. Ballonschulteranordnung (118; ... 1700) nach einem der vorhergehenden Ansprüche, wobei jede von der proximalen Ballonschulter (120; ... 1702) und der distalen Ballonschulter (122; ... 1704) befüllbar ist.

7. Ballonschulteranordnung (118; ... 1700) nach einem der vorhergehenden Ansprüche, wobei die proximale Ballonschulter (120; ... 1702) und die distale Ballonschulter (122; ... 1704) fließtechnisch nicht miteinander gekoppelt sind und individuell befüllbar sind.

8. Ballonschulteranordnung (118; ... 1700) nach einem der Ansprüche 1 bis 5, wobei jede von der proximalen Ballonschulter (120; ... 1702) und der distalen Ballonschulter (122; ... 1704) nicht befüllbar ist, wobei vorzugsweise jede von der proximalen Ballonschulter (120; ... 1702) und der distalen Ballonschulter (122; ... 1704) eine Wanddicke in einem Bereich von 0,000254 cm bis 0,0254 cm hat, und/oder wobei vorzugsweise der proximale Kragenabschnitt (216; ... 1108) ein offenes Ende hat, das zu einem offenen Ende des distalen Kragenabschnitts (222; ... 1116) weist.

9. Ballonschulteranordnung (118; ... 1700) nach einem der vorhergehenden Ansprüche, wobei jeder von dem proximalen Kragenabschnitt (216; ... 1108) und dem distalen Kragenabschnitt (222; ... 1116) trichterförmig mit einem weiteren Ende (230) ist, das sich von einem schmaleren Ende (232) radial auswärts aufweitet, wobei das schmalere Ende (232) des proximalen Kragenabschnitts (216; ... 1108) mit dem proximalen Schaftabschnitt (218; 318) verbunden ist, wobei das schmalere Ende (232) des distalen Kragenabschnitts (222; ... 1116) mit dem distalen Schaftabschnitt (224; 324) verbunden ist, und wobei die weiteren Enden (230) von jedem von dem proximalen Kragenabschnitt (216; ... 1108) und dem distalen Kragenabschnitt (222; ... 1116) zueinander weisen und normal zu der Zentralachse angeordnet sind.

10. Ballonschulteranordnung (118; ... 1700) nach einem der Ansprüche 1 bis 8, wobei jeder von dem proximalen Kragenabschnitt (216; ... 1108) und dem distalen Kragenabschnitt (222; ... 1116) eine Knollenform mit einem weiteren zentralen Abschnitt (340) hat, der an zwei entgegengesetzten Enden schmaler wird.

11. Ballonschulteranordnung (118; ... 1700) nach einem der Ansprüche 1 bis 8, wobei einer oder mehrere von dem proximalen Kragenabschnitt (216; ... 1108) und dem distalen Kragenabschnitt (222; ... 1116) einen zentralen zylindrischen Körper (810; 826; 910) einschließt/einschließen, der zwischen einem ersten zulaufenden Endabschnitt (812; 828; 912) und einem zweiten zulaufenden Endabschnitt (814; 830; 914) angeordnet ist, wobei der erste zulaufende Endabschnitt (812; 828; 912) und der zweite zulaufende Endabschnitt (814; 830; 914) in entgegengesetzte Richtungen von dem zentralen zylindrischen Körper (810; 826; 910) zu einem von dem zentralen Verbindungsabschnitt (214; ... 1306) oder einem entsprechenden von dem proximalen Schaftabschnitt oder dem distalen Schaftabschnitt (224; 324) zulaufen.

12. Ballonschulteranordnung (118; ... 1700) nach einem der Ansprüche 1 bis 8, wobei einer oder mehrere von dem proximalen Kragenabschnitt (216; ... 1108) und dem distalen Kragenabschnitt (222; ... 1116) einen gekrümmten zentralen Abschnitt einschließt/einschließen, der zwischen einem ersten zulaufenden Endabschnitt (812; 828; 912) und einem zweiten zulaufenden Endabschnitt (814; 830; 914) positioniert ist, wobei der erste zulaufende Endabschnitt (812; 828; 912) und der zweite zulaufende Endabschnitt (814; 830; 914) in entgegengesetzte Richtungen von dem zentralen Abschnitt (810; 826; 910) zu einem von dem zentralen Verbindungsabschnitt (214; ... 1306) oder einem entsprechenden von dem proximalen Schaftabschnitt oder dem distalen Schaftabschnitt (224; 324) zulaufen.

13. Ballonschulteranordnung (118; ... 1700) nach einem der Ansprüche 1 bis 8, wobei einer oder mehrere von dem proximalen Kragenabschnitt (1006) und dem distalen Kragenabschnitt (1022) einen länglichen zulaufenden Abschnitt (1010; 1026), einen kürzeren zulaufenden Abschnitt (1012; 1028) und einen zentralen Ringabschnitt (1014; 1030), der zwischen dem länglichen zulaufenden Abschnitt (1010; 1026) und dem kürzeren zulaufenden Abschnitt (1012; 1028) positioniert ist, einschließt/einschließen.

14. Verfahren zum Fertigen eines Ballonkatheters (100; 200), umfassend:
Blasformen einer Ballonschulteranordnung (118; ... 1700), die eine proximale Ballonschulter (120; ... 1702) und eine distale Ballonschulter (122; ... 1704) einschließt; und
Installieren der blasgeformten Ballonschulteranordnung (118; ... 1700) in dem Ballonkatheter (100; 200) durch Positionieren der Ballonschulteranordnung (118; ... 1700) innerhalb eines befüllbaren Hauptballons (108) des Ballonkatheters (100; 200).

15. Ballonkatheter (100; 200) für ein endovaskuläres Zuführsystem, umfassend:
eine proximale Ballonschulter (120; ... 1702), die auf einem Innenschaft des Ballonkatheters (100; 200) montiert ist, wobei die proximale Ballonschulter (120; ... 1702) einen komprimierbaren hohlen Mantel umfasst;
eine distale Ballonschulter (122; ... 1704), die auf dem Innenschaft montiert ist, wobei die distale Ballonschulter (122; ... 1704) einen komprimierbaren hohlen Mantel umfasst; und
einen befüllbaren Hauptballon (108), der die proximale Ballonschulter (120; ... 1702) und die distale Ballonschulter (122; ... 1704) umschließt.

## Revendications

1. Ensemble épaulement de ballonnet (118 ; 202 ; 302 ; 1100 ; 1300 ; 1500 ; 1600 ; 1700) destiné à un cathéter à ballonnet (100 ; 200), comprenant :
un épaulement de ballonnet proximal (120 ; 208 ; 308 ; 802 ; 1002 ; 1102 ; 1302 ; 1502 ; 1602 ; 1702) incluant une partie collier proximale (216 ; 316 ; 806 ; 1006 ; 1108) qui s'étend radialement vers l'extérieur à partir d'une partie tige proximale, par rapport à un axe central de l'ensemble épaulement de ballonnet (118 ; ... 1700) ; et
un épaulement de ballonnet distal (122 ; 212 ; 312 ; 804 ; 902 ; 1004 ; 1104 ; 1304 ; 1504 ; 1604 ; 1704) incluant une partie collier distale (222 ; 322 ; 1022 ; 1116) qui s'étend radialement vers l'extérieur à partir d'une partie tige distale (224 ; 324), chacun de l'épaulement de ballonnet proximal (120 ; ... 1702) et de l'épaulement de ballonnet distal (122 ; ... 1704) étant creux et comprenant un matériau moulé par soufflage compressible et étant conçu pour être positionné à l'intérieur du ballonnet principal gonflable (108) du cathéter à ballonnet (100 ; 200) et le supporter.

2. Ensemble épaulement de ballonnet (118 ; ... 1700) selon la revendication 1, des parois externes de l'épaulement de ballonnet proximal (120 ; ... 1702) et des parois externes de l'épaulement de ballonnet distal (122 ; ... 1704) étant conçues pour se comprimer ou s'étirer sous une pression appliquée et revenir à un état non comprimé ou non étiré lors du retrait de la pression appliquée.

3. Ensemble épaulement de ballonnet (118 ; ... 1700) selon l'une quelconque des revendications précédentes, la partie collier proximale (216 ; ... 1108) étant écartée de la partie collier distale (222 ; ... 1116), dans une direction axiale par rapport à l'axe central (220).

4. Ensemble épaulement de ballonnet (118 ; ... 1700) selon l'une quelconque des revendications précédentes, l'épaulement de ballonnet proximal (120 ; ... 1702) et l'épaulement de ballonnet distal (122 ;... 1704) étant moulés d'une seule pièce avec une partie de liaison centrale (214 ; 314 ; 820 ; 920 ; 1020 ; 1106 ; 1306), la partie de liaison centrale (214 ; ... 1306) étant agencée entre la partie collier proximale (216 ; ... 1108) et la partie collier distale (222 ;... 1116) et les reliant, de préférence, l'épaulement de ballonnet proximal (120 ; ... 1702) comprenant en outre une partie de transition proximale (226 ; 326) s'étendant entre la partie collier proximale (216 ; ... 1108) et la partie de liaison centrale (214 ; ... 1306) et les reliant, la partie de transition proximale se rétrécissant en diamètre à partir de la partie collier proximale (216 ; ... 1108) vers la partie de liaison centrale (214 ; ... 1306) et l'épaulement de ballonnet distal (122 ;... 1704) incluant en outre une partie de transition distale (228 ; 328) s'étendant entre la partie collier distale (222 ; ... 1116) et la partie de liaison centrale (214 ; ... 1306) et les reliant, la partie de transition distale se rétrécissant en diamètre à partir de la partie collier distale (222 ; ... 1116) vers la partie de liaison centrale (214 ;... 1306).

5. Ensemble épaulement de ballonnet (118 ; ... 1700) selon l'une quelconque des revendications 1 à 3, l'épaulement de ballonnet proximal (120 ; ... 1702) et l'épaulement de ballonnet distal (122 ; ... 1704) étant moulés séparément, en tant que deux pièces séparées.

6. Ensemble épaulement de ballonnet (118 ; ... 1700) selon l'une quelconque des revendications précédentes, chacun de l'épaulement de ballonnet proximal (120 ;... 1702) et de l'épaulement de ballonnet distal (122 ; ... 1704) étant gonflable.

7. Ensemble épaulement de ballonnet (118 ; ... 1700) selon l'une quelconque des revendications précédentes, l'épaulement de ballonnet proximal (120 ; ... 1702) et l'épaulement de ballonnet distal (122 ; ... 1704) n'étant pas accouplés fluidiquement l'un à l'autre et étant individuellement gonflables.

8. Ensemble épaulement de ballonnet (118 ; ... 1700) selon l'une quelconque des revendications 1 à 5, chacun de l'épaulement de ballonnet proximal (120 ; ... 1702) et de l'épaulement de ballonnet distal (122 ; ... 1704) n'étant pas gonflable, de préférence, chacun de l'épaulement de ballonnet proximal (120 ; ... 1702) et de l'épaulement de ballonnet distal (122 ; ... 1704) présentant une épaisseur de paroi dans une plage de 0,000254 cm à 0,0254 cm et/ou, de préférence, la partie collier proximale (216 ; ... 1108) présentant une extrémité ouverte faisant face à une extrémité ouverte de la partie collier distale (222 ; ... 1116).

9. Ensemble épaulement de ballonnet (118 ; ... 1700) selon l'une quelconque des revendications précédentes, chacune de la partie collier proximale (216 ; ... 1108) et de la partie collier distale (222 ; ... 1116) étant en forme d'entonnoir avec une extrémité plus large (230) qui s'évase radialement vers l'extérieur à partir d'une extrémité plus étroite (232), l'extrémité plus étroite (232) de la partie collier proximale (216 ; ... 1108) étant reliée à la partie tige proximale (218 ; 318), l'extrémité plus étroite (232) de la partie collier distale (222 ; ... 1116) étant reliée à la partie tige distale (224 ; 324) et les extrémités plus larges (230) de chacune de la partie collier proximale (216 ; ... 1108) et de la partie collier distale (222 ; ... 1116) se faisant face et étant agencées perpendiculairement à l'axe central.

10. Ensemble épaulement de ballonnet (118 ; ... 1700) selon l'une quelconque des revendications 1 à 8, chacune de la partie collier proximale (216 ; ... 1108) et de la partie collier distale (222 ; ... 1116) présentant une forme bulbeuse pourvue d'une partie centrale plus large (340) qui se rétrécit vers deux extrémités opposées.

11. Ensemble épaulement de ballonnet (118 ; ... 1700) selon l'une quelconque des revendications 1 à 8, une ou plusieurs de la partie collier proximale (216 ; ... 1108) et de la partie collier distale (222 ; ... 1116) incluant un corps cylindrique central (810 ; 826 ; 910) agencé entre une première partie d'extrémité effilée (812 ; 828 ; 912) et une seconde partie d'extrémité effilée (814 ; 830 ; 914), la première partie d'extrémité effilée (812 ; 828 ; 912) et la seconde partie d'extrémité effilée (814 ; 830 ; 914) s'effilant, dans des directions opposées, à partir du corps cylindrique central (810 ; 826 ; 910) vers l'une de la partie de liaison centrale (214 ; ... 1306) ou d'une partie correspondante parmi la partie tige proximale ou la partie tige distale (224 ; 324).

12. Ensemble épaulement de ballonnet (118 ; ... 1700) selon l'une quelconque des revendications 1 à 8, une ou plusieurs de la partie collier proximale (216 ; ... 1108) et de la partie collier distale (222 ; ... 1116) incluant une partie centrale incurvée positionnée entre une première partie d'extrémité effilée (812 ; 828 ; 912) et une seconde partie d'extrémité effilée (814 ; 830 ; 914), la première partie d'extrémité effilée (812 ; 828 ; 912) et la seconde partie d'extrémité effilée (814 ; 830 ; 914) s'effilant, dans des directions opposées, à partir de la partie centrale (810 ; 826 ; 910) vers l'une de la partie de liaison centrale (214 ; ... 1306) ou une partie correspondante parmi la partie tige proximale ou la partie tige distale (224 ; 324).

13. Ensemble épaulement de ballonnet (118 ; ... 1700) selon l'une quelconque des revendications 1 à 8, une ou plusieurs de la partie collier proximale (1006) et de la partie collier distale (1022) incluant une partie effilée allongée (1010 ; 1026), une partie effilée plus courte (1012 ; 1028) et une partie annulaire centrale (1014 ; 1030) positionnée entre la partie effilée allongée (1010 ; 1026) et la partie effilée plus courte (1012 ; 1028).

14. Procédé de fabrication d'un cathéter à ballonnet (100 ; 200), comprenant :
le moulage par soufflage d'un ensemble épaulement de ballonnet (118 ; ... 1700) incluant un épaulement de ballonnet proximal (120 ; ... 1702) et un épaulement de ballonnet distal (122 ; ... 1704) ; et
l'installation de l'ensemble épaulement de ballonnet (118; ... 1700) moulé par soufflage dans le cathéter à ballonnet (100 ; 200) par positionnement de l'ensemble épaulement de ballonnet (118 ; ... 17001) à l'intérieur d'un ballonnet principal gonflable (108) du cathéter à ballonnet (100 ; 200).

15. Cathéter à ballonnet (100 ; 200) pour un système de pose endovasculaire, comprenant :
un épaulement de ballonnet proximal (120 ; ... 1702) monté sur une tige interne du cathéter à ballonnet (100 ; 200), l'épaulement de ballonnet proximal (120 ; ... 1702) comprenant une coque creuse compressible ;
un épaulement de ballonnet distal (122 ; ... 1704) monté sur la tige interne, l'épaulement de ballonnet distal (122 ; ... 1704) comprenant une coque creuse compressible ; et
un ballonnet principal gonflable (108) qui renferme l'épaulement de ballonnet proximal (120 ; ... 1702) et l'épaulement de ballonnet distal (122 ; ... 1704).
